(19) Europäisches Patentamt
European Patent Office
Office européen des brevets

(11) Publication number: **0 334 956**
**A1**

(12)
# EUROPEAN PATENT APPLICATION
published in accordance with Art. 158(3) EPC

(21) Application number: 87906934.2

(22) Date of filing: 22.10.87

Data of the international application taken as a basis:

(86) International application number:
PCT/JP87/00810

(87) International publication number:
WO88/03027 (05.05.88 88/10)

(51) Int. Cl.³: A 61 K 35/14
A 61 L 31/00, A 61 M 5/00
A 01 N 1/02

(30) Priority: 22.10.86 JP 249552/86

(43) Date of publication of application:
04.10.89 Bulletin 89/40

(84) Designated Contracting States:
BE DE FR GB IT NL SE

(71) Applicant: Kao Corporation
14-10, Nihonbashi Kayabacho 1-chome
Chuo-Ku Tokyo 103(JP)

(84) Designated Contracting States:
BE DE FR GB IT NL SE

(71) Applicant: Terumo Kabushiki Kaisha
No. 44-1, Hatagaya 2-chome Shibuya-ku
Tokyo 151(JP)

(84) Designated Contracting States:
BE

(72) Inventor: NAGAI, H
Terumo K K TechnicalResearch and Development Div
2656-1, Ohbuchi, Fuji-shi Shizuoka 417(JP)

(72) Inventor: KUBOTA, Y
Terumo K K Technical Research and Development Div
2656-1, Ohbuchi, Fuji-shi Shizuoka 417(JP)

(72) Inventor: TAMURA, Y
Terumo K K Technical Research and Development Div
2656-1, Ohbuchi, Fuji-shi Shizuoka 417(JP)

(72) Inventor: SADO, M
Terumo K K Technical Research and Development Div
2656-1, Ohbuchi, Fuji-shi Shizuoka 417(JP)

(72) Inventor: KORA, S
Terumo K K Technical Research and Development Div
2656-1, Ohbuchi, Fuji-shi Shizuoka 427(JP)

(72) Inventor: KIMURA, Akio
727-103, Zenmyoji
Wakayama-shi, Wakayama 640(JP)

(72) Inventor: SUZUE, Shigetoshi
997-5, Meijin
Wakayama-shi Wakayama 640(JP)

(72) Inventor: MIYAMOTO, Norioki
1022, Hikata
Kainan-shi Wakayama 642(JP)

(74) Representative: Gillard, Marie-Louise et al,
Cabinet Beau de Loménie 55, Rue d'Amsterdam
F-75008 Paris(FR)

## (54) HEMOLYSIS INHIBITOR AND MEDICAL RESIN COMPOSITION, MEDICAL IMPLEMENT, AND BLOOD-PRESERVING FLUID CONTAINING THE SAME.

(57) A hemolysis inhibitor comprising a polycarboxylic acid ester, an ether compound and/or a monocarboxylic acid ester and a medical resin composition, medical implement, and blood-preserving fluid containing it. These compounds can prevent hemolysis of blood in contact therewith because of their erythrocytes-protecting activity.



Croydon Printing Company Ltd.

TITLE MODIFIED
see front page



DESCRIPTION

# HEMOLYSIS DEPRESSANTS AND MEDICAL RESIN COMPOSITIONS, MEDICAL IMPLEMENTS, AND BLOOD PRESERVING LIQUIDS USING SAID HEMOLYSIS DEPRESSANT

[Technical Field]

This invention relates to hemolysis depressants and to medical resin compositions, medical implements, and blood preserving liquid which use the hemolysis depressant. More particularly, it relates to hemolysis depressants which act with very high safety and provide highly effective control of the phenomenon of hemolysis which is liable to occur on the blood during the preservation thereof and to medical resin compositions obtained by incorporation of the hemolysis depressant, medical implements formed substantially of the medical resin composition, and blood preserving liquids obtained by incorporation of the hemolysis depressant.

[Background Art]

When blood flows out of the blood vessel, it begins to show a visible sign of coagulation within 10 to 20 minutes' exposure to the ambience. The clot which is formed by the coagulation is the final product of a series of chemical reactions which proceed during the conversion of fibrinogen into fibrin. The particles of the fibrin are interconnected and, during the course of the interconnection, erythrocytes are entrapped in the clot. In order for the blood for transfusion to retain its original liquid state, therefore, a measure to preclude the normal reactions responsible for the hemal coagulation must be taken during the extraction of the blood from a donor. Heretofore, the practice of adding a liquid anticoagulant as a blood preserving liquid to the freshly extracted blood has been in vogue. The liquid anticoagulant which is in popular use nowadays is intended to effect the preclusion of the

hemal coagulation by binding the element calcium which constitutes an important factor during the course of coagulation.

Incidentally, the blood for transfusion secured as described above is treated, when necessary, for separation of some of the components thereof and then is placed as in a blood bag, for example, to be preserved until use. When whole blood or a blood component such as concentrated red cells (CRC) is preserved for a long time, there ensues the so-called hemolysis, i.e. a phenomenon which involves fracture of erythrocytic membrane and consequent external liberation of hemoglobin. As main causes for the hemolysis, there can be cited the change in the pressure of osmosis due to a difference in the ion composition in the blood, the difference in the pressure of colloidal osmosis due to such proteinaceous components as hemoglobin, the change in membranous proteins and lipids of erythrocytes, the hindrance to the active transport of $Na^+$ and $K^+$, and the actions of medicines and poisons. The most important factor consists in the liquid anticoagulant to be used. Thus, numerous efforts have been devoted to improve the liquid anticoagulants. All the liquid anticoagulants currently in use are claimed to produce an anticoagulant action and an action for protection of erythrocytes as well. In the liquid anticoagulants of this type, however, the function as a protector for erythrocytes has not yet reached a point where it deserves to be called sufficient.

It is likewise important that the container such as the blood bag for preserving the extracted blood should be inactive relative to blood components. In the blood containers and other similar medical implements which are currently in use, those made of flexible vinyl chloride resin are predominant. The di-2-ethylhexyl phthalate (DOP) which is contained as a plasticizer in the flexible vinyl chloride resin for such medical implements possesses a large capacity for migration. It is known that when the flexible

vinyl chloride resin is exposed to blood, the di-2-ethylhexyl phthalate exudes from the container wall and dissolves into the blood (Medical Tool Science, 54, 221 (1984)). It has been reported that this di-2-ethylhexyl phthalate hinders the aggregating ability of platelets (Jouranl of Japan blood Transfusion Society, 28(3) 282 (1981)). When the preserving container made of such flexible vinyl chloride resin as described above is used to preserve blood, for example, there is the possibility that the di-2-ethylhexyl phthalate will enter the donee's blood vessel as entrained by the preserved blood during the course of transfusion. This possibility poses a problem from the standpoint of the adverse effect exerted by this compound upon the function of platelets.

For the solution of this problem, the feasibility of the use of a material not containing this di-2-ethylhexyl phthalate has been studied. It has been learnt that when blood is preserved in containers made of materials not containing di-2-ethylhexyl phthalate, the erythrocytes in the preserved blood are hemolyzed during the course of preservation. A search of the cause for this hemolysis has revealed that the absence of di-2-ethylhexyl phthalate, a substance effective in hindering hemolysis, is responsible for the hemolysis (Blood, 646 1270-(1984)). In other words, when blood is preserved in a blood container made of the conventional flexible vinyl chloride resin which incorporates therein di-ethylhexyl phthalate as a plasticizer, the di-2-ethylhexyl phthalate dissolving into the preserved blood serves to hinder the hemolysis of erythrocytes.

As a measure against such an adverse phenomenon as described above, a contradictory method which comprises preserving blood in a container made of a material incapable of exuding an incorporated plasticizer (or a material not containing any plasticier) and , prior to actual use of the preserved blood, adding di-2-ethylhexyl phthalate to the

POOR QUALITY



POOR QUALITY

blood thereby preventing the blood from hemolysis has been proposed (U.S. Patent No. 6,326,025). The idea of using di-2-ethylhexyl phthalate as a hemolysis depressant, however, is nothing desirable from the standpoint of physiological safety.

Further, the blood bag containing the blood extracted from a donor is generally forwarded to a hospital, as accompanied by a blood collection tube containing the same blood as that in the blood bag so that the blood in the tube may be used as a sample for testing the blood for compatibility to the patient's blood in advance of the transusion. As the time of storage of this blood bag increases to approach the 21st day of extraction fixed officially (in Japan) for the availability of concentrated red cells, the blood contained in the blood collection tube yields to hemolysis in much the same way as described above. The hemoglobin liberated in consequence of the hemolysis brings about adverse effects on the results of various measurements performed for clinical test and interferes with the test. This fact has posed as a problem.

As clearly gathered from the observations illustrated above, the desirability of developing a material capable of producing a notable protective action for erythrocytes has found enthusiastic recognition in various fields including the medical field.

An object of this invention, therefore, is to provide a novel hemolysis depressant and a medical resin composition, a medical implement and a blood preserving liquid which use the hemolysis depressant.

This invention also aims to provide a hemolysis depressant capable of providing highly effective control of the phenomenon of hemolysis which occurs in blood during the course of preservation. Further, this invention aims to provide a hemolysis depressant which excels also in physiological safety. This invention also aims to provide a

hemolysis depressant which can be incorporated in a resin composition or directly added to a liquid containing erythrocytes.

Further, this invention aims to provide a medical resin composition capable of very effectively controlling the phenomenon of hemolysis in a erythrocyte-containing liquid held in contact with the resin composition. This invention also aims to provide a flexible type medical resin composition which suffers from sparing (or substantially no) exudation of plasticizer, excels in physiological safety and produces a highly desirable effects in the inhibition of hemolysis, and suits best as a material for medical implements such as the blood bag. This invention further aims to provide a rigid type medical resin composition which produces a highly desirable effect in the inhibition of hemolysis and suits most as a material for such medical implements as the blood collection tube.

In addition, this invention aims to provide a medical implement capable of preserving blood components represented by the erythrocyte in a erythrocyte-containing liquid intact for a long time. This invention further aims to provide a medical implement abounding with physiological safety.

This invention further aims to provide a blood preserving liquid which possesses a highly desirable ability to preserve erythrocytes and abounds with safety. This invention also aims to provide a blood preserving liquid which permits protracted preservation of blood. Further, this invention aims to provide a blood preserving liquid which can be used as a liquid preserver in the anticoagulant and the additive system.

[Disclosure of the Invention]

The various objects of the present invention described above are accomplished by a hemolysis depressant which is made of one member or a mixture of two or more members selected from among the following compounds.



POOR QUALITY

(A) a carboxylic ester compound

i) possessing at least two ester bonds,

ii) having monovalent hydrocarbon groups coupled with the ester bonds, at least two of hydrocarbon groups being chain type hydrocarbon groups differing in number of carbon atoms,

iii) at least one of which monovalent hydrocarbon groups is a hydrocarbon group of not more than 3 or not less than 13 carbon atoms, providing that all of the monovalent hydrocarbon groups are not hydrocarbon groups of not more than 3 carbon atoms or hydrocarbon groups of not less than 13 carbon atoms, and

iv) at least one of which monovalent hydrocarbon groups possesses a branched structure, and

v) possessing a molecular weight of not more than 1,000;

(B) an ether compound possessing at least one ether bond and chain type monovalent hydrocarbon groups having 3 to 20 carbon atoms coupled with the ether oxygen, the molecular weight of the ether compound being not more than 1,000; and

(C) a monocarboxylic ester compound represented by the general formula(V):

$$RCOOR' \qquad (V)$$

wherein R and R' are independently a chain type hydrocarbon group of not less than 3 carbon atoms, providing that the total of the carbon atoms of R and R' falls in the range of 11 to 30.

This invention further discloses a hemolysis depressant wherein the aforementioned carboxylic ester compound (A) possesses two ester bonds. This invention also discloses a hemolysis depressant wherein the aforementioned carboxylic ester compound (A) is a dicarboxylic ester. This invention discloses a hemolysis depressant wherein at least one of the monovalent hydrocarbon groups coupled with the

ester bond of the aforementioned carboxylic ester compound is a hydrocarbon group of three carbon atoms and the other thereof is a hydrocarbon group of 8 to 20 carbon atoms. This invention further discloses a hemolysis depressant wherein the divalent hydrocarbon group in the acid moiety of the dicarboxylic ester is a hydrocarbon group of 2 to 12 carbon atoms. This invention also discloses a hemolysis depressant wherein the acid moiety of the dicarboxylic ester is maleic acid, succinic acid, glutaric acid, adipic acid, itaconic acid, sebacic acid, dodecandioic acid, or oxalacetic acid residue. Further, this invention discloses a hemolysis depressant wherein the aforementioned carboxylic ester compound (A) is selected from the group consisting of isopropyl 2-ethylhexyl maleate, isopropyldecyl maleate, isopropyl isotridecyl maleate, isopropyl tetradecyl maleate, and isopropyl myristyl maleate.

This invention discloses a hemolysis depressant wherein at least one of the monovalent hydrocarbon group coupled with the ether oxygen of the aforementioned ether compound (B) possesses a branched structure. This invention also discloses a hemolysis depressant wherein the aforementioned ether compound (B) possesses at least two ether bonds. This invention further discloses a hemolysis depressant wherein at least two of the monovalent hydrocarbon groups coupled with the ether oxygen have chains differing in length. This invention also discloses a hemolysis depressant wherein the aforementioned ether compound (B) is glycerine diether represented by the general formula (IV'):

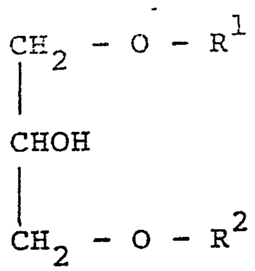

(IV')

wherein $R^1$ and $R^2$ are independently a chain type hydrocarbon group of 3 to 20 carbon atoms. Further, this invention discloses a hemolysis depressant wherein the glycerine diether represented by the general formula (IV') is glycerine-1-butyl-3-isostearyl ether or glycerine-1,3-bis(2-ethylhexyl) ether.

This invention discloses a hemolysis depressant wherein at least either of R and R' in the general formula (V) representing the monocarboxylic ester compound (C) possesses a branched structre. This invention further discloses a hemolysis depressant wherein the compound represented by the general formula (V) is isopropyl isolaurate, isopropyl oleate, 2-ethylhexyl isostearate, or 2-ethylhexyl 2-ethylhexanoate.

The various objects described above are accomplished by a flexible vinyl chloride type resin composition for medical use, characterized by comprising a vinyl chloride type resin, a plasticizer, and at least one hemolysis depressant selected from the group consisting of:

(A) a carboxylic ester compound

i) possessing at least two ester bonds,

ii) having monovalent hydrocarbon groups coupled with the ester bonds, at least two of hydrocarbon groups being chain type hydrocarbon groups differing in number of carbon atoms,

iii) at least one of which monovalent hydrocarbon groups is a hydrocarbon group of not more than 3 or not less than 13 carbon atoms, providing that all of the monovalent hydrocarbon groups are not

hydrocarbon groups of not more than 3 carbon atoms or hydrocarbon groups of not less than 13 carbon atoms, and

iv) at least one of which monovalent hydrocarbon groups possesses a branched structure, and

v) possessing a molecular weight of not more than 1,000;

(B) an ether compound possessing at least one ether bond and chain type monovalent hydrocarbon groups having 3 to 20 carbon atoms coupled with the ether oxygen, the molecular weight of the ether oxygen compound being not more than 1,000; and

(C) a monocarboxylic ester compound represented by the general formula(V):

RCOOR' (V)

wherein R and R' are independently a chain type hydrocarbon group of not less than 3 carbon atoms, providing that the total of the carbon atoms of R and R' falls in the range of 11 to 30.

This invention discloses a flexible vinyl chloride type resin composition for medical use wherein the plasticizer is incorporated in an amount in the range of 10 to 45% by weight and the hemolysis depressant is incorporated in an amount in the range of 1 to 30% by weight. This invention also discloses a flexible vinyl chloride type resin composition for medical use wherein the plasticizer possesses a low exuding property. This invention further discloses a flexible vinyl chloride type resin composition for medical use wherein the plasticizer is selected from the group consisting of trialkyl trimellitates, dinormal alkyl phthalates, and tetraalkyl pyromellitates. Further, this invention discloses a flexible vinyl chloride type resin composition for medical use wherein the plasticizer is dinormal decyl phthalate. This

invention also discloses a flexible vinyl chloride type resin composition for medical use wherein the plasticizer is trioctyl trimellitate.

The various objects described above are accomplished by a flexible resin composition for medical use, characterized by incorporating at least one hemolysis depressant selected from the group consisting of:

(A) a carboxylic ester compound

i) possessing at least two ester bonds,

ii) having monovalent hydrocarbon groups coupled with the ester bonds, at least two of hydrocarbon groups being chain type hydrocarbon groups differing in number of carbon atoms,

iii) at least one of which monovalent hydrocarbon groups is a hydrocarbon group of not more than 3 or not less than 13 carbon atoms, providing that all of the monovalent hydrocarbon groups are not hydrocarbon groups of not more than 3 carbon atoms or hydrocarbon groups of not less than 13 carbon atoms, and

iv) at least one of which monovalent hydrocarbon groups possesses a branched structure, and

v) possesing a molecular weight of not more than 1,000;

(B) an ether compound possessing at least one ether bond and chain type monovalent hydrocarbon groups having 3 to 20 carbon atoms coupled with the ether oxygen, the molecular weight of the ether compound being not more than 1,000; and

(C) a monocarboxylic ester compound represented by the general formula (V):

$$RCOOR' \qquad (V)$$

wherein R and R' are independently a chain type hydrocarbon group of not less than 3 carbon atoms, providing that the total of the carbon atoms of R and R' falls in the range of 11 to 30,

in a flexible resin composition containing no plasticizer.

This invention further discloses a flexible resin composition for medical use wherein the aforementioned hemolysis depressant is incorporated in an amount in the range of 5 to 35% by weight. This invention also discloses a flexible resin composition for medical use wherein the flexible resin is an internally plasticized vinyl chloride type resin, polyester, polyurethane, ethylene-vinyl acetate copolymer, or a polymer blend of polyvinyl chloride with polyurethane, an ethylenic polymer, or a caprolactone type polymer. Further, this invention discloses a flexible resin composition for medical use wherein the internally plasticized vinyl chloride type resin is urethane-vinyl chloride copolymer, vinyl acetate-vinyl chloride copolymer, or ethylene-vinyl acetate-vinyl chloride copolymer.

The various objects described above are accomplished by a rigid resin composition for medical use characterized by incorporating at least one hemolysis depressant selected from the group consisting of:

(A) a carboxylic ester compound
- i) possessing at least two ester bonds,
- ii) having monovalent hydrocarbon groups coupled with the ester bonds, at least two of hydrocarbon groups being chain type hydrocarbon groups differing in number of carbon atoms,
- iii) at least one of which monovalent hydrocarbon groups is a hydrocarbon group of not more than 3 or not less than 13 carbon atoms, providing that all of the monovalent hydrocarbon groups are not hydrocarbon groups of not more than 3 carbon atoms or hydrocarbon groups of not less than 13 carbon atoms, and
- iv) at least one of which monovalent hydrocarbon groups possesses a branched structure, and
- v) possessing a molecular weight of not more than 1,000;

(B) an ether compound possessing at least one ether bond and such chain type monovalent hydrocarbon groups having 3 to 20 carbon atoms coupled with the ether oxygen, the molecular weight of the ether compound being not more than 1,000; and

(C) a monocarboxylic ester compound represented by the general formula(V):

RCOOR' (V)

wherein R and R' are independently a chain type hydrocarbon group of not less than 3 carbon atoms, providing that the total of the carbon atoms of R and R' falls in the range of 11 to 30,

in a rigid resin composition.

This invention further discloses a rigid resin composition for medical use wherein the hemolysis depressant is incorporated in an amount in the range of 0.5 to 5% by weight. This invention also discloses a rigid resin composition for medical use wherein the rigid resin is selected from the group consisting of rigid vinyl chloride type resins, acrylic type resins, styrene type resins, olefin type resins, thermoplastic polyester type resins, and polycarbonates. This invention further discloses a rigid resin composition for medical use wherein the acrylic type resins are homopolymers or copolymers of methyl methacrylate, methyl acrylate, ethyl methacrylate, ethyl acrylate, acrylonitrile, and methacrylonitrile. Further, this invention discloses a rigid resin composition for medical use wherein the styrene type resins are polystyrene, acrylonitrile-styrene copolymer, and acrylonitrile-butadiene-styrene copolymer. This invention also discloses a rigid resin composition for medical use wherein the olefin type resins are polyethylene, polypropylene, and ethylene-propylene copolymer. This invention further discloses a rigid resin composition for

medical use wherein the thermoplastic polyester type resins are polyethylene terephthalate and polybutylene terephthalate.

The various objects described above are accomplished by a medical implement characterized by being substantially made of a flexible vinyl chloride type resin composition comprising a vinyl chloride type resin, a plasticizer, and at least one hemolysis depressant selected from the group consisting of:

(A) a carboxylic ester compound

i) possessing at least two ester bonds,

ii) having monovalent hydrocarbon groups coupled with the ester bonds, at least two of hydrocarbon groups being chain type hydrocarbon groups differing in number of carbon atoms,

iii) at least one of which monovalent hydrocarbon groups is a hydrocarbon group of not more than 3 or not less than 13 carbon atoms, providing that all of the monovalent hydrocarbon groups are not hydrocarbon groups of not more than 3 carbon atoms or hydrocarbon groups of not less than 13 carbon atoms, and

iv) at least one of which monovalent hydrocarbon groups possesses a branched structure, and

v) possessing a molecular weight of not more than 1,000;

(B) an ether compound possessing at least one ether bond and chain type monovalent hydrocarbon groups having 3 to 20 carbon atoms coupled with the ether oxygen, the molecular weight of the ether compound being not more than 1,000; and

(C) a monocarboxylic ester compound represented by the general formula(V):

$$RCOOR' \qquad (V)$$

wherein R and R' are independently a chain type hydrocarbon group of not less than 3 carbon atoms, providing that the total of the carbon atoms of R and R' falls in the range of 11 to 30.

This invention further discloses a medical implement which is a blood container substantially made of the aforementioned flexible vinyl chloride type resin composition. This invention also discloses a medical implement which is capable of withstanding the impacts of the sterilization carried out in an autoclave.

The various objects described above are accomplished by a medical implement characterized by being substantially made of a flexible resin composition incorporating at least one hemolysis depressant selected from the group consisting of:

(A) a carboxylic ester compound

i) possessing at least two ester bonds,

ii) having monovalent hydrocarbon groups coupled with the ester bonds, at least two of hydrocarbon groups being chain type hydrocarbon groups differing in number of carbon atoms,

iii) at least one of which monovalent hydrocarbon groups is a hydrocarbon group of not more than 3 or not less than 13 carbon atoms, providing that all of the monovalent hydrocarbon groups are not hydrocarbon groups of not more than 3 carbon atoms or hydrocarbon groups of not less than 13 carbon atoms, and

iv) at least one of which monovalent hydrocarbon groups possesses a branched structure, and

v) possessing a molecular weight of not more than 1,000;

(B) an ether compound possessing at least one ether bond and chain type monovalent hydrocarbon groups having 3 to 20 carbon atoms coupled with the ether oxygen, the molecular weight of the ether compound being not more than 1,000; and

(C) a monocarboxylic ester compound represented by the general formula(V):

RCOOR' (V)

wherein R and R' are independently a chain type hydrocarbon group of not less than 3 carbon atoms, providing that the total of the carbon atoms of R and R' falls in the range of 11 to 30,

in a flexible resin composition containing no plasticizer.

This invention further discloses a medical implement which is a blood container substantially made of the aforementioned flexible resin composition. This invention also discloses a medical implement which is capable of withstanding the impacts of the sterilization carried out in an autoclave.

The various objects described above are accomplished by a medical implement characterized by being substantially made of a rigid resin composition incorporating at least one hemolysis depressant selected from the group consisting of:

(A) a carboxylic ester compound

i) possessing at least two ester bonds,

ii) having monovalent hydrocarbons coupled with the ester bonds, at least two of hydrocarbon groups being chain type hydrocarbon groups differing in number of carbon atoms,

iii) at least one of which monovalent hydrocarbon groups is a hydrocarbon group of not more than 3 or not less than 13 carbon atoms, providing that all of the monovalent hydrocarbon groups are not

hydrocarbon groups of not more than 3 carbon atoms or hydrocarbon groups of not less than 13 carbon atoms, and

iv) at least one of which monovalent hydrocarbon groups possesses a branched structure, and

v) possessing a molecular weight of not more than 1,000;

(B) an ether compound possessing at least one ether bond and chain type monovalent hydrocarbon groups having 3 to 20 carbon atoms coupled with the ether oxygen, the molecular weight the ether compound being of not more than 1,000; and

(C) a monocarboxylic ester compound represented by the general formula(V):

RCOOR' (V)

wherein R and R' are independently a chain type hydrocarbon group of not less than 3 carbon atoms, providing that the total of the carbon atoms of R and R' falls in the range of 11 to 30,

in a rigid resin composition.

This invention discloses a medical implement which is a blood collection tube substantially made of the aforementioned rigid resin composition.

In addition, the various objects described above are accomplished by a blood preserving liquid characterized by incorporating therein at least one hemolysis depressant selected from the group consisting of:

(A) a carboxylic ester compound

i) possessing at least two ester bonds,

ii) having monovalent hydrocarbon groups coupled with the ester bonds, at least two of hydrocarbon groups being chain type hydrocarbon groups differing in number of carbon atoms,

iii) at least one of which monovalent hydrocarbon groups is a hydrocarbon group of not more than 3 or not less than 13 carbon atoms, providing that all of

POOR QUALITY

the monovalent hydrocarbon groups are not hydrocarbons of not more than 3 carbon atoms or hydrocarbon groups of not less than 13 carbon atoms, and

iv) at least one of which monovalent hydrocarbon groups possesses a branched structure, and

v) possessing a molecular weight of not more than 1,000;

(B) an ether compound possessing at least one ether bond and chain type monovalent hydrocarbon groups having 3 to 20 carbon atoms coupled with the ether oxygen, the molecular weight of the ether compound being not more than 1,000; and

(C) a monocarboxylic ester compound represented by the general formula(V):

RCOOR' (V)

wherein R and R' are independently a chain type hydrocarbon group of not less than 3 carbon atoms, providing that the total of the carbon atoms of R and R' falls in the range of 11 to 30.

This invention also discloses a blood preserving liquid which is an anticoagulant preservative liquid. This invention further discloses a blood preserving liquid containing as an additional component at least one compound selected from the group consisting of sodium citrate, citric acid, glucose, monosodium phosphate, adenine, sodium chloride, mannitol, maltose, sorbitol, multitol, sucrose, and lactose. This invention also discloses a blood preserving liquid incorporating the aforementioned hemolysis depressant in a substratal liquid selected from the group consisting of ACD liquid, CPD liquid, CPDA-1 liquid, CPDA-2 liquid, SAG liquid, and SAG liquid incorporating therein mannitol, maltose, multitol, sorbitol, sucrose, or lactose. This invention further discloses a blood preserving liquid

incorporating therein the aforementioned hemolysis depressant in a filnal concentration in the range of 100 μM to 10 mM, preferably 400 μM to 4 mM.

[Brief Description of the Drawings]

Fig. 1 is a front view illustrating a blood bag as a typical medical implement embodying the present invention,

Fig. 2 is a cross section illustrating a blood collection tube as another typical medical implement embodying the present invention, and

Figs. 3 and 4 are cross sections illustrating the another embodiment which is put to use.

[Best Mode for Carrying Out the Invention]

The hemolysis depressant of the present invention is made of one member or a mixture of two or more members selected from the group consisting of:

(A) a carboxylic ester compound

i) possessing at least two ester bonds,

ii) having monovalent hydrocarbon groups coupled with the ester bonds, at least two of hydrocarbon groups being chain type hydrocarbon groups differing in number of carbon atoms,

iii) at least one of which monovalent hydrocarbon groups is a hydrocarbon group of not more than 3 or not less than 13 carbon atoms, providing that all of the monovalent hydrocarbon groups are not hydrocarbon groups of not more than 3 carbon atoms or hydrocarbon groups of not less than 13 carbon atoms, and

iv) at least one of which monovalent hydrocarbon groups possesses a branched structure, and

v) possessing a molecular weight of not more than 1,000;

(B) an ether compound possessing at least one ether bond and chain type monovalent hydrocarbon groups having 3 to 20 carbon atoms coupled with the ether oxygen, the molecular weight of the ether compound being not more than 1,000; and (C) a monocarboxylic ester compound represented by the general formula(V):

RCOOR' (V)

wherein R and R' are independently a chain type hydrocarbon group of not less than 3 carbon atoms, providing that the total of the carbon atoms of R and R' falls in the range of 11 to 30.

Surprisingly, it has been found that the carboxylic ester compound (A), the ether compound (B), and the monocarboxylic ester compound (C) produce the same action to prevent erythrocytes from hemolysis as di-2-ethylhexyl phthalate and, meanwhile, unlike di-2-ethylhexyl phthalate, produce no discernible action to inhibit any ability of platelet aggregation and that they offer high safety.

Furhter, these compounds are invariably capable of being dispersed in erythrocyte-containing liquids and are capable of being uniformly dispersed therein particularly when they are in the form of emulsions. Thus, they can be added directly to such erythrocyte-containing liquids. Since these compounds possess thorough compatibility with various synthetic resins represented by vinyl chloride type resins, they are enabled to function as a hemolysis depressant when they are incorporated in synthetic resin compositions. For example, when a synthetic resin composition incorporating therein a carboxylic ester compound (A), an ether compound (B), and /or a monocarboxylic ester compound (C) is exposed to contact with an erythrocyte-containing liquid, the compounds exude from the resin composition and pass into the

erythrocyte-containing liquid and, therefore, bring about the same effect as when they are directly added to the erythrocyte-containing liquid.

Further, in the embodiment which resides in incorporating the carboxylic ester compound (A), the ether compound (B), and/or the monocarboxylic ester compound (C) in a flexible vinyl chloride type resin composition, the effect to be brought about by this embodiment can be further enhanced by additionally incorporating therein a plasticizer other than di-2-ethylhexyl phthalate, particularly a plasticizer of the foregoing description which possesses high safety and a low exuding property. To be more specific, when the aforementioned flexible vinyl chloride type resin composition is exposed to contact with blood, the hemolysis of erythrocytes is prevented by the action of the carboxylic ester compound (A), the ether compound (B), and/or the monocarboxylic ester compound (C) exuding from the resin composition and passing into the blood and, in the meantime, the resin composition does not liberate through exudation any substance which inhibits the ability to coagulate platelets similarly to di-2-ethylhexyl phthalate. This resin composition, therefore, excels in physiological safety and in the ability to protect erythrocytes. The same thing applies to the embodiment which resides in incorporating the caoboxylic ester compound (A), the ether compound (B), and/or the monocarboxylic ester compound (C) in a flexible resin composition containing no plasticizer. (This is because the problem essentially due to the exudation of a plasticizer cannot arise in the present embodiment.) For this reason, the flexible vinyl chloride type resin composition for medical use and the flexible resin composition for medical use contemplated by the present invention are suitable as materials for medical implements. Further, the shaped articles formed by using these materials, owing to the excellence thereof in safety, fabricability, flexibility, transparency, and thermal

resistance, are enabled to manifest the effect thereof to the fullest extent when the shaped articles are used as medical implements. The effect is prominent particularly when the shaped articles are medical implements such as the blood bag destined to contact body fluids such as blood.

Similarly, in the embodiment which resides in incorporating the carboxylic ester compound (A), the ether compound (B), and/or the monocarboxylic ester compound (C) in a rigid resin composition, the compounds function to prevent the hemolysis of erythrocytes and offer highly satisfactory protection for erythrocytes. The resin composition, therefore, is suitable as a material for medical implements. The shaped articles formed by using this material, owing to their excellence in safety, fabricability, transparancy, and thermal resistance, are enabled to manifest the effect to the fullest extent when the shaped articles are used as medical implements. The effect is prominent particularly when the shaped articles are medical implements such as the blood collection tubes which are destined to contact body fluids such as blood.

The aforementioned carboxylic ester compound (A), the ether compound (B), and/or the monocarboxylic ester compound (C) are capable of being dispersed in the form of emulsion or clathrate compound in an aqueous solution. Further, they exhibit the aforementioned ability to prevent the hemolysis of erythrocytes even when they are in the form mentioned above. When they are incorporated in a blood preserving liquid with the aid of a suitable surfactant which is incapable of exerting any adverse effect upon blood components or an alpha-cyclodextrin, the produced blood preserving liquid excels in the ability to protect erythrocytes as well as in physiological safety. When the blood preserving liquid of this invention is added to an erythrocyte-containing liquid such as whole blood or concentrated red cells, for example, most of the erythrocytes can be retained for a long time in the same

state as immediately after blood extraction. Thus, the problem arising in the transfusion of preserved blood can be eliminated.

Now, this invention will be described more specifically below with reference to embodiments thereof. For the purpose of facilitating comprehension of the present invention, the sections titled "Hemolysis Depressant," "Resin composition for Medical Use," "Medical Implement," "Blood Preserving Liquid," and "Example" will be included in the following part of the text of specification.

Hemolysis Depressant

The hemolysis depressant of the present invention is made of one member or a mixture of two or more members selected from the group consisting of carboxylic ester compounds (A), ether compounds (B), and monocarboxylic ester compounds (C).

The hemolysis depressant of the present invention can be used as directly added to the erythrocyte-containing liquid such as whole blood or concentrated red cells (inclusive of the case wherein the addition is made to an erythrocyte-containing liquid contained in advance in a blood container such as the blood bag in combination with an anticoagulant preservative liquid such as ACD (acid-citrate dextrose) or CPD (citrate phosphate dextrose) or it can be used as incorporated in a synthetic resin composition for medical use to form blood containers such as the blood bag and the blood collection tube and medical implements such as the catheter, transfusion set, and blood circuits which are destined to contact an erythrocyte-containing liquid.

In the direct addition of the hemolysis depressant of this invention to the erythrocyte-containing liquid, while the compounds to be described in detail later on may be added in their unmodified form, they are added more desirably in the form of emulsion or clathrate compound so as to be uniformly dispersed and mixed in the erythrocyte-containing liquid. The preparation of the

hemolysis depressant in the form of emulsion can be attained by dispersing the hemolysis depressant in a suitable aqueous medium such as a physiological salt water or buffer or in an anticoagulant preservative such as ACD liquid or CPD liquid with the aid of a surfactant or curing castor oil which has no adverse effect upon blood components as illustrated in the following section titled "Hemolysis Preserving Liquid." The preparation of the hemolysis depressant in the form of clathrate compound is attained by the use of -cyclodextrin, for example. The amount of the homolysis depressant to be added directly to the erythrocyte-containing liquid, though variable with the kind of the erythrocyte-containing liquid and the kind of the compound used to manifest the action to preclude the hemolysis, falls in the range of 100 μM to 10 mM, preferably 300 μM to 5 mM, as final concentration based on the amount of whole blood.

In the incorporation of the hemolysis depressant of the present invention in a synthetic resin composition for medical use, since the compounds to be described in detail later on can be uniformly dispersed and mixed in various resin compositions such as, for example, vinyl chloride type resins, olefin type resins, styrene type resins, (meth)acryl type resins, and carbonate type resins, it suffices to add the hemolysis depressant to the synthetic resin composition while the resin composition is being blended. The synthetic resin composition which has incorporated the hemolysis depressant as described above can be formed in a given shape by any of the conventional molding methods such as, for example, calender molding, extrusion molding, injection molding, and plastisol molding. The adhesion of the resin composition to a surface can be attained by any of the conventional methods such as, for example, high-frequency welding, thermal welding and supersonic welding. When the hemolysis depressant of the present invention is incorporated in one of such various

resin composition, the hemolysis depressant which exudes from the synthetic resin composition and comes into contact with the erythrocyte-containing liquid to manifest the action to protect erythrocytes. The amount of the hemolysis depressant to be incorporated in the synthetic resin composition, though variable with the kind of the synthetic resin composition used to incorporate the hemolysis depressant and the kind of the compound expected to manifest the aforementioned action, is generally in the range of 0.5 to 35% by weight based on the amount of the resin composition. So long as the amount of the hemolysis depressant so incorporated falls in the aforementioned range, it brings about the action to protect erythrocytes effectively when the erythrocyte-containing liquid is brought into contact with the medical implement to be formed with the aforementioned synthetic resin composition. The hemolysis depressant so incorporated does not substantially impair the physical properties of the synthetic resin composition.

Carboxylic ester compound (A)

The carboxylic ester compound which has an ability to inhibit hemolysis as contemplated by the present invention fulfils the requirement that it should:

i) possess at least two ester bonds,

ii) have monovalent hydrocarbon groups coupled with the ester bonds, at least two of hydrocarbon groups being chain type hydrocarbon groups differing in number of carbon atoms,

iii) at least one of which monovalent hydrocarbon groups is a hydrocarbon group of not more than 3 or not less than 13 carbon atoms, providing that all of the monovalent hydrocarbon groups are not hydrocarbon groups of not more than 3 carbon atoms or hydrocarbon groups of not less than 13 carbon atoms, and



POOR QUALITY

iv) at least one of which monovalent hydrocarbon groups possesses a branched structure, and

v) possess a molecular weight of not more than 1,000.

The carboxylic ester compounds according to this invention embrace both the ester compounds of polycarboxylic acids with monohydric alcohols and the ester compounds of monocarboxylic acids with polyhydric alcohols to be described more fully later on, condition that they satisfy the requirment, i) through v), indicated above. This invention prefers the ester compounds of polycarboxylic acids with monohydric alcohols to the other ester compounds mentioned above. The carboxylic ester compound of the foregoing description is subject to the requirement that at least two of the monovalent hydrocarbon groups coupled with the ester bond should possess chains differing in length. This requirement is essential because the carboxylic ester compound fails to acquire the ability to inhibit hemolysis if the hydrocarbon groups possess chains of one and the same length. It is further subject to the requirement that at least one of the aforementioned monovalent hydrocarbon groups is a hydrocarbon group of not more than 3 carbon atoms or a hydrocarbon group of not less than 13 carbon atoms and all the monovalent hydrocarbon groups are not hydrocarbon groups of not more than 3 carbon atoms or hydrocarbon groups of not less than 13 carbon atoms. This requirement is essential because the carboxylic ester compound on being incorporated in the resin composition, no longer possesses compatibility with the resin or acquires an ability to protect erythrocytes if all the monovalent hydrocarbon groups have not more than 3 carbon atoms or not less than 13 carbon atoms. It is further because the ability of platelet aggregation is liable to be impaired if all the monovalent hydrocarbon groups have not less than 4 and not more than 12 carbon atoms. Further, the requirement that at least one of the aforementioned monovalent hydrocarbon groups should possess a branched structrue is

essential because the compatibility of the carboxylic ester compound is deficient in compatibility with the resin and in ability to inhibit hemolysis. The carboxylic ester compound requires to possess a molecular weight of not more than 1,000. This requirement is essential because the carboxylic ester compound assumes a solid state, fails to retain compatibility with the resin, acquires uniform dispersion in the resin composition only with difficulty, and experiences difficulty to acquire sufficient dispersion in the erythrocyte-containing liquid if the molecular weight exceeds 1,000.

When the carboxylic ester compound is to be produced with a polycarboxylic acid and a monohydric alcohol, the acid moiety thereof is the residue of a chain type polycarboxylic acid of 2 to 12 carbon atoms, preferably 4 to 6 carbon atoms such as, for example, oxalic acid, malonic acid, succinic acid, glutaric acid, adipic acid, maleic acid, fumaric acid, itaconic acid, alpha-ketoglutarilic acid, oxaloacetic acid, isocitric acid sebasic acid, or dodecandioic acid and the alcohol moiety thereof is the residue of a chain type monohydric alcohol of 1 to 22 carbon atoms, preferably 1 to 20 carbon atoms, such as, for example, methyl alcohol, ethyl alcohol, a propyl alcohol, a butyl alcohol, an amyl alcohol, a hexyl alcohol, a heptyl alcohol, an octyl alcohol, a nonyl alcohol, a decyl alcohol, an undecyl alcohol, a lauryl alcohol, a tridecyl alcohol, a myristyl alcohol, a pentadecyl alcohol, a palmityl alcohol, a heptadecyl alcohol, a stearyl alcohol, or a behenyl alcohol. Where the carboxylic ester compound is produced with a polycarboxylic acid and a monohydric alcohol as described above, the polyvalent hydrocarbon group in the acid moiety formed of a polycarboxylic acid residue may be a (branched or linear) saturated hydrocarbon group or an (branded or linear) unsaturated hydrocarbon group. Optionally, the hydrocarbon group may contain a hydroxyl group as a characteristic

POOR QUALITY

group. Similarly, the monovalent hydrocarbon group in the alcohol moiety formed of a monohydric alcohol residue may be a saturated hydrocarbon group or an unsaturated hydrocarbon group.

When the carboxylic ester compound of the foregoing description is made with a polyhydric alcohol and a monocarboxylic acid, the alcohol moiety thereof is the residue of a polyhydric alcohol of 2 to 12 carbon atoms, preferably 2 to 6 carbon atoms, such as, for example ethylene glycol, diethylene glycol, triethylene glycol, tetraethylene glycol, propylene glycol, trimethylene glycol, dipropylene glycol, 1,2-butane diol, 1,3-butane diol, 1,4-butane diol, 2,3-butane diol, or glycerine and the acid moiety thereof is the residue of a fatty acid of 2 to 20 carbon atoms, preferably 2 to 18 carbon atoms, such as, for example, acetic acid, propionic acid, isopropionic acid, butyric acid, isobutyric acid, valeric acid, isovaleric acid, pivalic acid, caproic acid, isocarproic acid, enanthic acid, isoheptaic acid, caprylic acid, isocaprylic acid, 2-ethylhexylanoic acid, pelargonic acid, isopelargonic acid, capric acid, isocapric acid, undecylic acid, isoundecylic acid, lauric acid, isolauric acid, tridecylic acid, isotridecylic acid, myristic acid, isomyristic acid, pentadecylic acid, isopentadecylic acid, palmitic acid, isopalmitic acid, heptadecylic acid, isoheptadecylic acid, stearic acid, isostearic acid, nonadecanoic acid, isononadecanoic acid, arachic acid, isoarachic acid, acrylic acid, crotonic acid, isocrotonic acid, undecyleic acid, oleic acid, elaidic acid, sorbic acid, linolic aicd, linolenic acid, or arachidonic acid. Where the carboxlic ester compound is made of a polyhydric alcohol and a monocarboxylic acid as described above, the polyvalent hydrocarbon group in the alcohol moiety formed of a polyhydric alcohol residue may be a (branched or linear) saturated hydrocarbon or an (branched or linear) unsaturated hydrocarbon group. Optionally, this hydrocarbon group may

contain an ether oxygen as a characteristic group. Similarly, the monovalent hydrocarbon group in the acid moiety made of a monocarboxylic acid residue may be saturated hydrocarbon group or an unsaturated hydrocarbon group.

In the carboxylic ester compounds of the foregoing description, those possessing two carboxylic ester bonds and preferably dicarboxylic esters are cited as particularly excelling in the effect of controlling hemolysis. In the group of these compounds, those in which one of the monovalent hydrocarbon groups coupled with the ester bonds is a hydrocarbon group of 3 carbon atoms and the other monovalent hydrocarbon groups are those of 8 to 20 carbon atoms manifest the effect conspicuously. Further in the case of the dicarboxylic esters, the divalent hydrocarbon groups in the acid moiety are desired to be hydrocarbon groups of 2 to 12 carbon atoms such as, for example, the residues of maleic acid, succinic acid, glutaric acid, adipic acid, itaconic acid, sebacic acid, dodecandioic acid, and oxalacetic acid. Most desirable are maleic ester compounds represented by the general formula (I):

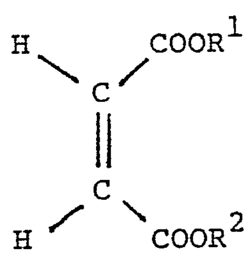

(I)

wherein $R^1$ is a chain type hydrocarbon group of 1 to 3, preferably 3, carbon atoms, $R^2$ is a chain type hydrocarbon group of 8 to 20, preferably 8 to 16, carbon atoms, providing that at least either of $R^1$ and $R^2$ possesses a branched structure.

Typical desirable examples of the compound represented by the general formula (I) include isopropyl-2-hexyl maleate, isopropyl decyl maleate, isopropyl isotridecyl maleate, isopropyl tetradecyl maleate, and isopropyl myristyl maleate.

Ether compound (B)

The ether compound which has an ability to inhibit hemolysis as contemplated by the present invention fulfils the requirement that it should possess at least one ether bond, and chain type monovalent hydrocarbon groups having 3 to 20, preferably 4 to 18, carbon atoms coupled with the ether oxygen, the a molecular weight of the ether compound being not more than 1,000.

The ether compound is subject to the requirement that the monovalent hydrocarbon groups coupled with the ether oxygen should be severally chain type hydrocarbon groups of 3 to 20 carbon atoms. This requirement is essential because the ether compound possesses no ability to inhibit hemolysis if the chain type hydrocarbon groups possess less than 3 carbon atoms or because the ether compound assumes a solid state, fails to exhibit compatibility with the resin on being incorporated in a resin composition, acquires uniform dispersion in the resin composition only with difficulty, and experiences difficulty to acquire sufficient dispersion in the erythrocyte-containing liquid if the chain type hydrocarbon groups possess more than 20 carbon atoms. The ether compound is further subject to the requirement that it should possess a molecular weight of not more than 1,000. This requirement is essential because the ether compound similarly assumes a solid state, fails to retain compatibility with the resin, acquires uniform dispersion in the resin composition only with difficulty, and experiences difficulty to acquire sufficient dispersion in the erythrocyte-containing liquid if the molecular weight exceeds 1,000. In the ether compound, at least one of the

monovalent hydrocarbon groups coupled with the ether bond is desired to possess a branched structure for the purpose of improving the compatibitliy with the resin and enhancing the effect of inhibiting hemolysis. In order for the ether compound to manifest a still better effect in the inhibition of hemolysis, the ether compound is desired to possess at least two ether bonds and, where two or more ether bonds are present, at least two of the monovalent hydrocarbon groups coupled with the ether oxygens possess chains differing in length. The monovalent hydrocarbon groups coupled with the ether oxygens in this ether compound may be chain type saturated hydrocarbon groups or unsaturated hydrocarbon groups.

As typical examples of the ether compounds described above, there can be cited those represented by the general formula (II):

$$R^1 - O - R^2 \qquad (II)$$

wherein $R^1$ and $R^2$ are independently chain type hydrocarbon groups of 3 to 20 carbon atoms, those represented by the general formula (III):

$$R^1 - O \leftarrow\!\!\left(R^4\ O\right)\!\!\rightarrow_n R^2 \qquad (III)$$

wherein $R^1$ and $R^2$ are independently chain type hydrocarbon groups of 3 to 20 carbon atoms, $R^4$ stnads for a linear or branched saturated hydrocarbon group of 1 to 3 carbon atoms, and n stands for an integer in the range of 1 to 5, and those represented by the general formula (IV):

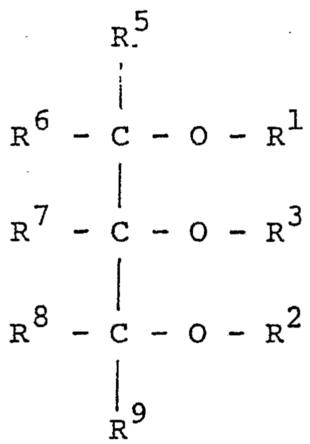

(IV)

wherein $R^1$ through $R^3$ are independently chain type hydrocarbon groups of 3 to 20 carbon atoms or hydrogen atom and $R^5$ through $R^9$ are independently a methyl group or hydrogen atom, providing that at least two of the substituents, $R^1$ through $R^3$, are not both hydrogen atom. Of course, these are not limitative examples.

More specifically, examples of the compound represented by the general formula (II) include 2-ethylhexyl isopropyl ether, di-2-ethylhexyl ether, diisopentyl ether, diisolauryl ether, diisomyristyl ether, diisopalmityl ether, and diisostearyl ether. Examples of the compound represented by the general formula (III) include ethylene glycol diethers such as ethylene glycol di(2-ethylhexyl) ether, ethylene glycol 2-ethylhexyl isopropyl ether, ethylene glycol isopentyl isopropyl ether, ethylene glycol isopentyl butyl ether, ethylene glycol isolauryl isopropyl ether, ethylene glycol isolauryl butyl ether, ethylene glycol isomyristyl isopropyl ether, ethylene glycol isomyristyl butyl ether, ethylene glycol isopalmityl isopropyl ether, ethylene glycol isopalmityl butyl ether, ethylene glycol isostearyl isobutyl ether, and ethylene glycol isostearyl butyl ether, diethylene glycol diethers such as diethylene glycol di(2-ethylhexyl) ether, diethylene glycol 2-ethylhexyl isopropyl ether, diethylene glycol isopentyl isopropyl ether, diethylene glycol isopentyl butyl ether, diethylene glycol isolauryl isopropyl ether,

diethylene glycol isolauryl butyl ether, diethylene glycol isomyristyl isopropyl ether, diethylene glycol isomyristyl butyl ether, diethylene glycol isopalmityl isopropyl ether, diethylene glycol isopalmityl butyl ether, diethylene glycol isostearyl isopropyl ether, and diethylene glycol isostearyl butyl ether, propylene glycol diethers such as propylene glycol di(2-ethylhexyl) ether, propylene glycol 2-ethylhexyl isopropyl ether, propylene glycol isopentyl isopropyl ether, propylene glycol isopentyl butyl ether, propylene glycol isolauryl isopropyl ether, propylene glycol isolauryl butyl ether, propylene glycol isomyristyl isopropyl ether, propylene glycol isomyristyl butyl ether, propylene glycol isopalmityl isopropyl ether, propylene glycol isopalmityl butyl ether, propyleme glcyol isostearyl isopropyl ether, and propylene glycol isostearyl butyl ether, similar triethylene glycol diethers, dipropylene glycol diethers, and butylene glycol diethers. Examples of the compound represented by the general formula (IV) include glycerine diethers such as glycerine-1,3-bis(2-ethylhexyl) ether, glycerine-1-isopropyl-3-(2-ethylhexyl) ether, glycerine-1-isopropyl-3-isopentyl ether, glycerine-1-butyl-3-isopentyl ether, glycerine-1-isopropyl-3-isolauryl ether, glycerine-1-butyl-3-isolauryl ether, glycerine-1-isopropyl-3-isomyristyl ether, glycerine-1-butyl-3-isomyristyl ether, glycerine-1-isopropyl-3-isopalmityl ether, glycerine-1-butyl-3-isopalmityl ether, glycerine-1-isopropyl-3-isostearyl ether, and glycerine-1-butyl-3-isostearyl ether and glycerine triethers such as glycerine triisopropyl ether, glycerine triisobutyl ether, glycerine triisopentyl ether, 1,3-diisopropoxy-2-(2-ethylhexyloxy) propane, 1,3-bis-(ethylhexyloxy)-2-isopropoxy propane, 1,3-dibutoxy -2-(2-ethylhexyloxy) propane, 1,3-bis-(2-ethylhexyloxy) -2-butoxy propane, 1,3-diisopropoxy-2-isopentyloxy propane, and 1,3-diisopentyloxy-2-butoxy propane. Of these ether compounds enumerated above, particularly desirable are glycerine diethers represented by the general formula (IV'):

$$\begin{array}{l} CH_2 - O - R^1 \\ | \\ CHOH \\ | \\ CH_2 - O - R^2 \end{array} \qquad (IV')$$

wherein $R^1$ and $R^2$ are independently a chain type hydrocarbon group of 3 to 20 carbon atoms. Especially desirable ether compounds are glycerine-1-butyl-3-isostearyl ether and glycerine-1,3-bis(2-ethylhexyl) ether.

Monocarboxylic ester compound (C)

The monocarboxylic ester compound which has an ability to inhibit hemolysis as contemplated by the present invention fulfils the requirement that it should be a compound represented by the general formula (V):

$$RCOOR' \qquad (V)$$

wherein R and R' independently stand for a chain type hydrocarbon group of not less than 3, more desirably 3 to 22, and most desirably 3 to 18, carbon atoms, providing that the total of the carbon atoms of R and R' falls in the range of 11 to 30, preferably 11 to 21.

The monocarboxylic ester compound represented by the general formula (V) is subject to the requirement that the numbers of carbon atoms in the subtituents, R and R', should not be less than 3. This requirement is essential because the compound is liable to manifest toxicity and, therefore, is unsuitable for various applications which demand inhibition of hemolysis if the chain type hydrocarbon group possesses less than 3 carbon atoms. Further, the monocarboxylic ester compound represented by the general formula (V) is subject to the requirement that the total of carbon atoms of R and R' should fall in the range of 11 to 30. This requirement is essential because the monocarboxylic ester compound assumes no ability to inhibit hemolysis if the total of carbon atoms is less than 11 or because the compound fails to exhibit compatibility with the resin on being incorporated in a resin composition, acquires

uniform dispersion in the resin composition only with difficulty, and experiences difficulty to acquire uniform dispersion in the erythrocyte-containing liquid if the total of carbon atoms exceeds 30. Further, in order for the monocarboxylic ester of the general formula (V) to be improved in comatibility with the resin and in the ability to inhibit hemolysis, at least either of the substituents, R and R', in the chain type hydrocarbon group is desired to possess a branched structure. The chain type hydrocarbon groups, R and R', may be saturated chain type hydrocarbon groups or unsaturated chain type hydrocarbon groups. Specifically, as examples of the monocarboxylic ester represented by the general formula (V), include there can be cited 2-ethylhexyl butyrate, 2-ethylhexyl isobutyrate, isoheptyl valerate, 2-ethylhexyl valerate, isoheptyl pivalate, 2-ethylhexyl pivalate, isopentyl hexanoate, isohexyl hexanoate, 2-ethylhexyl hexanoate, isobutyl 2-ethylhexanoate, isopentyl 2-ethylhexanoate, 2-ethylhexyl 2-ethylhexanoate, isopropyl decanoate, isobutyl decanoate, 2-ethylhexyl decanoate, isopropyl laurate, isobutyl laurate, 2-ethylhexyl laurate, 2-ethylhexyl isolaurate, isopropyl myristate, isobutyl myristate, 2-ethylhexyl myristate, isopropyl palmitate, isobutyl palmitate, 2-ethylhexyl palmitate, isopropyl stearate, isobutyl stearate, 2-ethylhexyl stearate, isopropyl isostearate, isobutyl isostearate, 2-ethylhexyl isostearate, isopropyl oleate, isobutyl oleate, and 2-ethylhexyl oleate. Of course, these are not exclusive examples. Among other compounds enumerated above, isopropyl isolaurate, isopropyl oleate, 2-ethylhexyl isosterate, and 2-ethylhexyl 2-ethylhexanoate prove to be paricularly desirable. Most desirable of all these compounds is isopropyl isolaurate.

<u>Resin Composition for Medical Use</u>

The resin composition contemplated for medical use by the present invention is obtained by incorporating in a varying synthetic resin composition at least one hemolysis

depressant selected from the group consisting of carboxylic ester compounds (A), ether compounds (B), and monocarboxylic ester compounds (C).

As described above, the carboxylic ester compounds (A), the ether compounds (B), and the monocarboxylic ester compounds (C) possess compatibility with various synthetic resins and can be uniformly dispersed in various flexible and rigid synthetic resin compositions. A carboxylic ester compound (A), an ether compound (B), and/or a monocarboxylic ester compound (C) incorporated in a resin composition for medical use is in such quality that, when the synthetic resin composition comes into contact with an erythrocyte-containing liquid, the compound exudes from the resin composition and passes into the liquid and acts to protect the erythrocytes. The resin composition for medical use according to this invention, therefore, is most suitable as a material for medical implements such as the blood bag and the blood collection tube which are destined to contact blood. The resin composition for medical use may be prepared as flexible products or rigid products, depending on purposes for which the products are adopted. In various resin composition, flexible vinyl chloride type resin compositions may be cited as most desirable. When a carboxylic ester compounds (A), an ether compound (B), and/or a monocarboxylic ether compound (C) is incorporatd in a flexible vinyl chloride type resin composition, the inhibition of hemolysis in a erythrocyte-containing liquid is effected by the compound which exudes from the resin composition and passes into the liquid. Thus, the resin composition is allowed to use a plasticizer other than di-2-ethylhexyl phthalate, particularly a plasticizer of the description which exhibits high physiological safety and a low exuding property.

<u>Flexible vinyl chloride type resin composition for medical use</u>

The flexible vinyl chloride type resin composition for medical use according to this invention is characterized by a composition which comprises a vinyl chloride type resin, a plasticizer, and at least one hemolysis depressant selected from the group consisting of carboxylic ester compounds (A), ether compounds (B), and monocarboxcylic ester compounds (C).

As the vinyl chloride resin to be used in the flexible vinyl chloride type resin composition contemplated by this inveniton for medical use, there can be used vinyl chloride homopolymer, polyvinylidene chloride, or a varying copolymer containing not less than 40% by weight, more desirably not less than 65% by wight, and most desirably not less than 75% by weight, of vinyl chloride in addition to the balance of other copolymerizable monomer. The average polymerization degree of the vinyl chloride resin is in the range of 400 to 3,000, more desirably 600 to 2,700, and most desirably 800 to 1,700. Examples of the comonomer for vinyl chloride in the copolymer include vinylidene chloride, ethylene, propylene, vinyl acetate, vinyl bromide, vinyl fluoride, styrene, vinyl toluene, vinyl pyridine, acrylic acid, alkyl acrylate (such as methyl acrylate, ethyl acrylate, isopropyl acrylate, n-butyl acrylate, and 2-ethylhexyl acrylate), methacrylic acid, alkyl methacrylates (such as methyl methacrylate, ethyl methacrylate, and 2-ethylhexyl methacrylate), acrylonitrile, and methacrylonitrile. Optionally the vinyl chloride type resin may incorporate therein styrene-acrylonitrile copolymer or styrene-methacrylonitrile copolymer.

The plasticizer to be used in the flexible vinyl chloride type resin composition contemplated for medical use by the present invention is desired to possess high physiological safety and preferably exhibit a low exuding property. As examples of the plasticizer meeting this description, there can be cited dinormal alkyl phthalates such as dinormal octyl phthalate, dinormal nonyl phthalate,



POOR QUALITY

dinormal decyl phthalate, dinormal undecyl phthalate, and dilauryl phthalate, trialkyl trimellitates such as tri-2-ethylhexyl trimellitate and trinormal octyl trimetllitate, and tetraalkyl pyromellitates such as tetra-2-ethylhexyl pyromellitate. Among other plasticizers enumerated above, dinormal decyl phthalate and trioctyl trimellitate prove to be particularly desirable. In the flexible vinyl chloride type resin composition for medical use according to the present invention, the plasticizer of the foregoing description is incorporated in an amount generally in the range of 10 to 45% by weight, preferably 15 to 35% by weight, though variable with the kind of the plasticizer to be used. This range is important because the produced resin composition lacks flexibility if the amount of the plasticizer incorporated is less than 10% by weight or because the composition is apt to induce the phenomenon of bleeding if the amount of the plasticizer exceed 45% by weight.

Then, in the flexible vinyl chloride type resin composition contemplated for medical use by this invention, the hemolysis depressant selected from the group consisting of carboxylic ester compounds (A), ether compounds (B), and monocarboxylic ester compounds (C) is incorporated. As the hemolysis depressant, one compound selected from the aforementioned group of compounds or two or more compounds similarly selected from the group can be used. This hemolysis depressant is incorporated in a concentration in the range of 1 to 30% by weight, preferably 3 to 15% by weight in the flexible vinyl chloride type resin composition for medical use contemplated by this invention. This range is important because the action to inhibit hemolysis of erythrocytes is not sufficient if this concentration is less than 1% by weight or because the incorporated hemolysis depressant possibly impairs the physical properties of the flexible vinyl resin type composition if the concentration exceeds 30% by weight.

Further, the flexible vinyl chloride type resin composition for medical use according to the present invention, when necessary, may incorporate therein an epoxidized vegetable oil such as epoxidized soy bean oil or epoxidized linseed oil intended as a combination stabilizer and auxiliary plasticizer; a metallic soap of calcium or zinc with stearic acid, lauric acid, ricinoleic acid, or naphthenic acid intended as a stabilizer; a lubricant; an antioxidant; etc. The amount of the epoxidized vegetable oil to be additionally incorporated as a combination stabilizer and auxiliary plasticizer is in the range of 3 to 10% by weight, preferably 6 to 9% by weight and the amount of the metallic soap to be incorporated as a stabilizer is in the range of 0.05 to 3% by weight, preferably 0.1 to 1.5% by weight.

When the vinyl chloride type resin composition for medical use according to the present invention is to be formed in a desired shape, this formation can be accomplished by any of the conventional methods such as, for example, calender molding, extrusion molding, and plastisol molding methods. When the resin composition is to be joined to a surface, the adhension can be effected by high frequency welding, thermal welding, or supersonic welding, etc.

Flexible resin composition for medical use

The homolysis depressant of the present invention can be incorporated as effectively in any flexible resin composition than the flexible vinyl chloride resin composition as in the vinyl chloride resin composition, to give rise to a highly desirably flexible resin composition for medical use.

The flexible resin composition contemplated for medical use by the present invention, therefore, is characterized by incorporating at least one hemolysis depressant selected from the group consisting of carboxylic

ester compounds (A), ether compounds (B), and monocarboxylic ester compounds (C) which have been defined above into said flexible resin composition containing no plasticizer.

The flexible resin to be used in the manufacture of the flexible resin composition for medical use is a flexible resin which exhibits sufficient plasticity, particularly flexibility, without being externally plasticized with a plasticizer (providing that the term "plasticizer" as used in the present specificaiton refers, unless otherwise defined particularly, to an external plasticizer in the narrow sense of the word). The flexible resin composition for medical use, therefore, contains no plasticizer. This means that this flexible resin composition for medical use is inherently incapable of entailing as a problem the disadvantage that a plasticizer exudes from the resin composition and exerts an adverse effect on blood components or on the vital system.

As examples of the flexible resin of the foregoing description, there can be cited internally plasticized vinyl chloride type resins, polyethylene, thermoplastic polyester, polyurethane, ethylene-vinyl acetate copolymer, and polymer blends of polyvinyl chloride with polyurethane, ethylenic type polymers (such as, for example, a product marketed under trademark designation of "ERBAROY"), or caprolactone type polymers. Of course, these are not exclusive examples. Examples of the internally plasticized vinyl chloride resin include urethane-vinyl chloride copolymer, vinyl acetate-vinyl chloride copolymer, and ethylene-vinyl acetate-vinyl chloride copolymer. In the internally plasticized vinyl chloride type resin of this nature, the gravimetric ratio of the vinyl chloride monomer component to the monomer component bestowed with a plasticizing capacity and used for copolymerization falls in the range of about 7 : 3 to 3 : 7, preferably 6 : 4 to 4 : 6. The polyethylene for use in the copolymerization is desired to be a low-density polyethylene preferably possessing a melt index

in the range of about 0.1 to 5. The thermoplastic polyester for the copolymerization is typified by a polyethylene terephthalate film. Typical examples of the polyurethane include polyester type polyurethane and polyether type polyurethane elastomers. Preferably, the polyurethane is a polyether type segmented polyurethane. In the ethylene-vinyl acetate copolymer, the gravimetric ratio of the ethylene monomer component to the vinyl acetate component is approximately in the range of 95 : 5 to 70 : 30, preferably 90 : 10 to 80 : 20.

This invention does not discriminate the flexible resin composition for medical use particularly on account of the kind of flexible resin component. Among other various available flexible resin components, polyurethane and ethylene-vinyl acetate copolymer can be cited as particularly desirable flexible resins components.

In the flexible resin composition of this invention for medical use, the aforementioned hemolysis depressant to be selected from the group consisting of carboxylic ester compounds (A), ether compounds (B), and monocarboxylic ester compounds (C) defined above is incorporated. As the hemolysis depressant, one compounds selected from the aforementioned group of compounds or two or more compounds similarly seleted from the group can be incorporated. The homolysis depressant thus selected is incorporated in the flexible resin composition contemplated for medical use by this invention in a concentration in the ragne of 5 to 35% by weight, preferably 10 to 25% by weight. This range is important because the produced composition manifests no sufficient action to inhibit hemolysis of erythrocytes if the concentration is less than 5% by weight or because the incorporated hemolysis depressant possibly impair the physical properties of the flexible resin composition if the concentration exceeds 35% by weight.

Optionally, the flexible resin composition of this invention for medical use may incorpoate therein such additives as stabilizer, lubricant, and antioxidant.

When the flexible resin composition of this invention for medical use is to be formed in a given shape, this formation can be effected by any of the various conventional methods available for any flexible resin composition, such as calender molding, extrusion molding, blow molding, and plastisol molidng. When the flexible resin composition is to be joined to a surface, the adhesion can be effected by high frequency welding, thermal welding, or supersonic welding, etc., depending on the kind of the flexible resin.

Rigid resin composition for medical use

The rigid resin composition of the present invention for medical use is characterized by incorporating at least one hemolysis depressant selected from the group consisting of carboxylic ester compounds (A), ether compounds (B), and monocarboxylic ester compounds (C) defined above into said rigid resin composition.

No particular limits are imposed on the rigid resin to be used in the rigid resin composition contemplated for medical use by this invention, except for the sole requirement that this rigid resin should be physiologically safe and properly compatible with a carboxylic ester cmpound (A), an ether compound (B), and/or a monocarboxylic ester compound (C) defined above. As examples of the rigid resin answering the description, there can be cited rigid vinyl chloride type resins, acrylic type resins, styrene type resins, olefinic type resins, thermoplastic polyester type resins, and polycarbonate. The rigid resin described above is desired, without reference to the kind thereof, to possess a melt flow index exceeding 0.5 g/10 min., preferably falling in the range of 5 to 12 g/10 min. Examples of the rigid vinyl chloride type resin include a homopolymer of vinyl chloride and copolymers containing not

less than 40 % by weight, more desirably not less than 65 % by weight, and most desirably not less than 75 % by weight, of vinyl chloride and the balance of other copolymerizable monomer such as vinylidene chloride, ethylene, propylene, styrene, methyl methacrylate, or acrylonitrile. Examples of the acrylic type resin include homopolymers and copolymers of alkyl (meth)acrylates such as methyl methacrylate, methyl acrylate, ethyl methacrylate, and ethyl acrylate, acrylonitrile, and methacrylonitrile. Examples of the polystyrene type resin include polystyrene, acrylonitrile-styrene copolymer, and acrylonitrile-butadiene-styrene copolymer (ABS resin). Examples of the olefinic type resin include medium- to high-density polyethylenes, polypropylene, and copolymers by the combination of ethylene, propylene, and other -olefins such as ethylene-propylene copolymer. Examples of the thermoplastic polyester type resin are polyethylene terephthalate and polybutylene terephthalate. Examples of the polycarbonate include bisphenol-A type polycarbonate and polycarbonates possessing various carbonate ester type structures. Of course, the rigid resin to be used in the rigid resin composition contemplated for medical use by this invention is not limited to those enumerated above as examples. Polymer blends of various resins are also examples of the rigid resin usable herein.

In the rigid resin composition of the present invention for medical use, the aforementioned hemolysis depressant to be selected from the group consisting of carboxylic ester compounds (A), ether compounds (B), and monocarboxylic ester compounds (C) defined above is incorporated. As the hemolysis depressant, one compound selected from the aforementioned group of components or two or more members selected similarly from the group can be used. In the rigid resin composition of this invention for medical use, the hemolysis depressant is incorporated in a concentration in the range of 0.5 to 5% by weight,

preferably 2 to 3% by weight. This range is important because the produced composition fails to manifest a sufficient action to inhibit hemolysis if the concentration of the hemolysis depressant is less than 0.5% by weight or because the incorporated hemolysis depressant possible impairs the physical properties of the rigid resin composition if the concentration exceeds 5% by weight.

Optionally, the rigid resin composition of the present invention for medical use may incorporate therein additives such as stabilizer, lubricant, and antioxidant.

When the rigid resin composition of this invention for medical use is to be formed in a given shape, this formation can be effected by any of the conventional methods available for any rigid resin composition such as, for example, injection molding and extrusion molding.

Medical Implement

The medical implement of the present invention is formed substantially of the flexible vinyl chloride type resin composition for medical use, the flexible resin composition for medical use, or the rigid resin composition for medical use described above.

To be specific, the medical implement of the present invention is characterized by being made substantially of a flexible vinyl chloride type resin composition comprising a vinyl chloride type resin, a plasticizer, and at least one hemolysis depressant selected from the group consisting of carboxylic ester compounds (A), ether compounds (B), and monocarboxylic ester cmpounds (C) defined above.

The medical implement is excellent in various properties such as safety, processibility, flexibility, and thermal resistance and particularly in the ability to inhibit hemolysis of erythrocytes.

The medical implement which constitutes another aspect of this invention is characterized by being made substantially of a flexible resin composition incorporating

at least one hemolysis depressant selected from the group consisting of carboxylic ester compounds (A), ether compounds (B), and monocarboxylic ester compounds (C) defined above into said flexible resin composition containing no plasticizer. This medical implement is excellent in various properties such as safety, processibility, flexibility, and thermal resistance and particularly in the ability to inhibit hemolysis of erythrocytes.

Concrete examples of the medical implement which is made of the flexible vinyl chloride type resin composition for medical use or the flexible resin composition for medical use according to the present invention include blood containers such as blood bag, catheter, transfusion sets, and blood circuits which are destined to contact blood or other body fluids. Containers for packaging such medical implements as mentioned above and containers for prepared agents such as tablets are other examples.

Now, the medical implement of the present invention as embodied in the form of a blood bag will be described below with reference to the accompanying drawing. Fig. 1 illustrates the blood bag. A blood collection bag 3 which is made of the aforementioned flexible vinyl chloride type resin composition or the aforementioned flexible resin composition and provided with a plurality of outlets 1 fitted with peel tab and a connecting outlet 2 has the periphery 4 thereof heat sealed by high frequency heating or some other suitable heating means. This blood collection bag 3 has connected thereto a blood collection tube 6 made of the aforementioned flexible vinyl chloride type resin composition or the aforementioned flexible resin composition and adapted to communicate with an inner space 5 of the blood collection bag 3. A piercing needle 8 is fixed in a needle base 7 formed at the leading end of the blood collection tube 6. This piercing needle 8 is sheathed in a



POOR QUALITY

cap 9. To the connecting outlet 2 of the aforementioned blood collection bag 3 is joined a connection tube 17 through the medium of a connection needle 16 formed at the leading end thereof. A connection tube 13 provided with an outlet 10 fitted with a peel tab, made of the aforementioned flexible vinyl chloride type resin composition or the aforementioned flexible resin composition, and adapted to communicate with an inner space 12 of a first satellite bag 14 made of the aforementioned flexible vinyl chloride type resin composition or the aforementioned flexible resin composition and having the periphery thereof similarly heat sealed is caused to communicate with the connection tube 17 via a branched tube 15. Further, a connection tube 22 made of the aforementioned flexible vinyl chloride type resin composition or the aforementioned flexible resin composition and adapted to communicate with an inner space 20 of a second satellite bag 23 provided with an outlet 18 fitted with a peel tab, made of the aforementioned flexible vinyl chloride type resin composition or the aforementioned flexible resin composition, and having the periphery thereof similarly heat sealed is caused to communicate with the connection tubes 17 and 13 via the branched tube 15.

This three-component blood bag is capable of separating collected blood into components in a closed system. From the piercing needle 8 plunged into the vein of a donor, a prescribed amount of blood is drawn into the blood collection bag 3 via the blood collection tube 6. After the extraction of blood is completed, the blood collection bag 3 is subjected to centrifugal force to have the blood separated into an upper layer of platelet rich plasma and a lower layer of hemocytes. Then, the platelet rich plasma of the upper layer is forced out of the blood collection bag 3 and tranferred via the connection tubes 17 and 13 to the first satellite bag 14. The first satellite bag 14 now containing the platelet rich plasma is further subjected to centrifugal force so as to have the platelet

rich plasma separated into an upper layer of platelet concentrate and a lower layer of platelet poor plasma. The platelet concentrate of the upper layer is forced out of the first satellite bag 14 and transferred via the connection tubes 13 and 22 into the second satellite bag 23. Even when the collected blood is exposed to contact with the blood bags and the tubes for a long time as by being centrifugally separated into components, distributed into pertinent blood bags and then stored, the aforementioned flexible vinyl chloride type resin composition or the aforementioned flelxible resin composition of which the blood bags and the tubes are made is excellent in the effect of protecting erythrocytes and incapable of interfering with the aggregating ability of platelets and, therefore, permits safe and efficient transfusion of components of blood.

This invention has been so far described as embodied in a blood bag. Other medical implements such as containers for body fluids, catheters, transfusion sets, and blood circuits, containers for packaging the aforementioned medical implements, and containers for prepared agents such as tablets can be similarly advantageously made with the aforementioned flexible vinyl chloride type resin composition or the aforementioned flexible resin composition.

These medical implements by nature are subjected to sterilization prior to use. This sterilization is effected with ethylene oxide or in an autoclave. The sterilization by use of an autoclave is adopted preferably. In the autoclave, the medical implements is sterilized generally at 121°C for about 60 minutes. The medical implements of the present invention, as described above, possesses ample thermal stability to endure the sterilizing conditions used in the autoclave.

The medical implements which constitutes yet another aspect of this invention is characterized by being made substantially of a rigid resin composition

incorporating at least one hemolysis depressant selected from carboxylic ester compounds (A), ether compound (B), and monocarboxylic ester compounds (C) defined above in said rigid resin composition. This medical implement is excellent in various physical properties such as processibility and thermal resistance and particularly in the ability to inhibit hemolysis of erythrocyltes.

Particularly desirable examples of the medical implement of this invention which is made of the aforementioned rigid resin composition for medical use defined above are blood collection tubes which are exposed, particularly for a long time, to contact with body fluids or solutions of body fluid components such as blood or concentrated red cells. Other than these blood collection tubes, desirable examples of the medical implement include blood collection vials, test tubes, petri dishes, and housings for various artificial organs such as artificial lungs and artificial kidneys and heat exchangers. Of course, these are not exclusive examples.

Now, the medical implements of the present invention as embodied in the form of a blood collection tube will be described below with reference to the accompanying drawing. Fig. 2 illustrates a vacuum blood collection tube 31 which comprises a tubular member 32 closed at one end and opened at the other end thereof and made of the rigid resin composition for medical use according to the present invention and a pierceable stopper member 34 made of butyl rubber and adapted to close airtighly an open end 33 of the aforementioned tubular member 32 so as to keep an inner space 35 in a vacuumized state. The vacuum blood collection tube 31 which is constructed as described above is put to use as follows. As illustrated in Fig. 3, the vacuum blood collection tube 31 is inserted, with the aforementioned open end 33 in the lead, into a blood collection tube holder 38 opened at one end and closed at the other end thereof, made of the rigid resin composition for medical use according to

the present invention, and provided with a blood collection needle 37 helically fitted into a threaded hole 36 formed in a closed end 40. The blood collection needle 37 is enclosed with a luer adapter 39 made of synthetic resin and formed of a blood vessel piercing part 37a and a stopper piercing part 37b. When the blood vessel piercing part 37a of the blood collection needle 37 is forcibly inserted into the closed end 40 of the blood collection tube holder 38, the stopper piercing part 37b of the blood collection needle 37 pierces the luer adapter 39 and the stopper member 34 and reaches the inner space 35 of the blood colloection tube 31 to establish communication between the blood vessel and the aforementioned inner space 35, with the result that the blood inside the blood vessel, owing to the negative pressure in the inner space 35, is caused to flow into the inner space of the blood collection tube 31 in a total amount corresponding to the prevailing degree of vacuum. The extraction of the blood is terminated by removing the blood vessel piercing part 37b of the blood collection needle 37. The blood thus collected is preserved inside the blood collection tube 31 until it is subjected to test. Since the blood collection tube 31 is made of the rigid resin composition for medical use according to this invention as described above, the hemolysis depressant which exudes from the composition and passes into the blood acts to inhibit hemolysis of erythrocytes. Even when the preservation of the blood protracted much in duration, therefore, various measurements made for clinical test are not adversely affected by free homoglobin. Thus, the measurements are allowed to produce accurate results.

This invention has been so far described as embodied in the form of a blood collection tube. Other medical implements such as blood collection vials, test tuebes, petri dishes, and housings for various artificial

organs such as artificial lungs and kidneys and heat exchangers can be made similarly advantageously with the aforementioned rigid resin composition.

Blood Preserving Liquid

The blood preserving liquid of the present invention is characterized by having incorporated therein a hemolysis depressant selected from the group consisting of carboxlic ester compounds (A), ether compounds (B), and monocarboyxlic ester compounds (C) defined above. As the hemolysis depressant, one compound selected from the group of compounds mentioned above or two or more compounds similarly selected from the group can be used.

The carboxylic ester compounds (A), ether compunds (B), and monocarboxylic ester compounds (C) mentioned above are invariably capable of being dispersed in an aqueous solution by the use of a suitable surfactant or alpha-cyclodextrin which exerts no adverse effect on blood components. Even when they are so dispersed, they still retain the aforementioned ability to inhibit hemolysis of erythrocytes. When a blood preserving liquid incorporates such a compound therein, it is enabled to acquire a notable ability to protect erythrocytes while retaining highly desirable physiological safety. By the addition of the blood preserving liquid to an erythrocyte-containing liquid such as whole blood or concentrated red cells, the greater part of the erythrocytes are allowed to retain for a long time the same state as immediately after extraction of blood. Thus, the problem heretofor encountered in the trasfusion of preserved blood can be eliminated.

Uniform dispersion of a carboxylic ester compound (A), an ether compound (B), and/or a monocarboyxlic ester compound (C) defined above in the blood preserving liquid of the present invention can be advantageously accomplished by preparatorily preparing the compound in the form of an emulsion by the use of a surfactant incapable of exerting any adverse effect on blood components or a curing castor

oil or in the form of a clathrate compound by the use of alpha-cyclodextrin, for example. As concrete examples of the surfactant, there can be cited polyoxyethylene sorbitan monoesters (represented by Tween series) such as polyoxyethylene sorbitan monolaurate (Tween 20), polyoxyethylene sorbitan monopalmitate (Tween 40), polyoxyethylene sorbitan monostearate (Tween 60), and polyoxyethylene sorbitan monooleate (Tween 80), polyoxyethylene polyoxypropylene block copolymers (represented by products of BASF marketed under trademark designation of "Pluradot HA-430"), and sorbitan monoacyl esters (represented by Span series) such as sorbitan monoacyl laurate (Span 20), sorbitan monoacyl palmitate (Span 40),sorbitan monoacyl stearate (Span 60), and sorbitan monoacyl oleate (Span 80). Of course, the amount of the surfactant or α-cyclodextrin to be added for this purpose is desired to be on the minimum level tolerable from the standpoint of physiological safety. The ether compound, even when it is added in the form of an emulsion or a clathrate compound, possesses the ability to protect erythrocytes.

The hemolysis depressant selected from the group consisting of carboxylic ester compounds (A), ether compound (B), and monocarboxylic ester compound (C) defined above behaves in such a manner, though depending on the kind of the hemolysis depressant used, in the blood preserving liquid of the present invention that is produces the ability to protect erythrocytes conspicuously when it is incorporated in the preserved blood in a final concentration in the range of 100μM to 10mM, preferably 400μM to 4mM.

The other components to be contained in the blood preserving liquid of the present invention include the same components as those contained in the conventional blood preserving liquid such as the anticoagulant preservative which is added to whole blood collected or the conventional blood cell preserving liquid intended for addition to

concentrated red cells in the additive system. Concrete examples of these components are sodium citrate, citric acid, glucose, monosodium phosphate, adenine, sodium chloride, mannitol, maltose, sorbitol, multitol, sucrose, and lactose. To be specific, the blood preserving liquid of the present inveniton is desried to be prepared by using as a basic liquid an anticoagulant preservative liquid such as, for example, ACD solution (acid citrate dextrose anticoagulant solution), CPD solution (citrate phosphate dextrose anticoagulant solution), CPDA-1 solution (citrate phosphate dextrose (1.25 x CPD) plus 0.25 mM adenine), or CPDA-2 solution (citrate phosphate dextrose (1.75 x CPD) plus 0.50 mM adenine), or a blood cell preserving liquid such as, for example, SAG solution (saline-adenine-glucose solution), or a modified SAG solution additionally incorporating therein mannitol, maltose, multitol, sorbitol, sucrose, or lactose (Japanese Patent Provisional Publication No. 139,419/81) and incorporating the carboxylic ester compound (A), the ether compound (B) and/or the monocarboxylic ester compound (C) defined above in the basic liquid. Of course, the blood preserving liquid of the present invention is not limited in any sense to the composition mentioned above. It is only required to incorporated the carboxylic ester compound (A), the ether compond (B) and/or the monocarboxlic ester compound (C) defined above and to have a composition safe physiologically.

Actual use of the blood preserving liquid of this invention may be effected by directly adding this liquid to the blood or by placing this liquid in advance in the medical implement such as blood bag and allowing it to be mixed with the blood when the blood is introduced into the implement.

<u>Example</u>

Now, a number of working examples will be cited herein below to facilitate comprehension of the present invention. These examples are cited solely for the purpose of illustration of this invention and are not meant to limit the scope of this invention in any sense.

Example 1

Isopropyl isotridecyl maleate was added to a 2000μg/ml polyoxyethylene monooleate (reagent-grade product of Wako Junyaku K.K. marketed under trademark designation of "Tween 80")/physiological saline mixed solution in such an amount to be contained therein in a final concentration of 4mM. A 0.2ml aliquot of the resultant emulsion was added to a human concentrated red cells (hereinafter referred to as "CRC") adjusted in advance to a hematocrit value of about 70% so as to be contained therein in a final concentration indicated in Table 1. The resulting liquid was placed in a stoppered polypropylene tube and left standing at rest at 4°C for 4 weeks. At the end of this standing, it was tested for plasma hemoglobin concentration by the TMB method (Clin. Chem., 23, 749-(1977)). The results are shown in Table 1.

Example 2

An emulsion was prepared by following the procedure of Example 1, except that isopropyl decyl maleate was used in place of isopropyl isotridecyl maleate. A CRC sample incorporating the emulsion was left standing and thereafter tested for plasma hemoglobin concentration. The results are shown in Table 1.

Conctrols 1 and 2

Emulsions were prepared by following the procedure of Excample 1, except that di-2-ethylhexyl phthalate (Control 1) and diisopropyl maleate, a carboxylic ester compound deviating from the scope of the present invention (Control 2) were respectively used in place of isopropyl isotridecyl maleate. CRC samples severally incorporating

the emulsions were left standing and, thereafter, tested for plasma hemoglobin concentration. The results are shown in Table 1.

Control 3

A CRC sample incorporating a 2000 μg/ml polyoxyethylene monooleate (reagent-grade product of Wako Junyasku K.K. marketed under tardemark designation of "Tween 80")/ phsiological saline water mixed solution alone was left standing and thereafter, tested for plasma hemglobin concentration. The results are shown in Table 1.

Examples 1 and 2 and Controls 1 through 3 are experiments which were carried out on one and the same CRC.

Table 1

| | Reagent | Final concent-ration (mM) | Hemoglobin concentration in plasma (Mg/ml) |
|---|---|---|---|
| Example 1 | Isopropyl isotridecyl maleate | 4.0<br>0.4 | 33<br>35 |
| Example 2 | Isopropyl decyl maleate | 4.0<br>0.4 | 43<br>41 |
| Control 1 | Di-2-ethylhexyl phthalate | 0.5 | 41 |
| Control 2 | Diisopropyl maleate | 4.0<br>0.4 | 138<br>124 |
| Control 3 | - | - | 123 |

Example 3

Isopropyl isotridecyl maleate was added to CRC adjusted in advance to a hematocrit value of about 70% so as to be contained threrein in a final concentration indicated in Table 2 and stirred gently. The resultant liquid was placed in a stoppered polypropylene tube and left standing at rest at 4°C for 4 weeks, while subjecting to gentle

stirring per a week. Thereafter, it was tested for plasma hemoglobin concentration by following the procedure of Example 1. The results are shown in Table 2.

Example 4

By following the procedrue of Example 3, isopropyl decyl maleate was added to CRC in the place of isopropyl isotridecyl maleate. The resultant liquid was left standing and, thereafter, tested for plasma hemoglobin concentration. The results are shown in Table 2.

Controls 4 and 5

CRC samples were obtained by following the procedure of Example 3, except that di-2-ethylhexyl phthalate (Control 4) and diisopropyl maleate, a carboxylic ester compound deviating from the scope of this invention, (Control 5) were respectively used in place of isopropyl isotridecyl maleate. The resultant liquids were left standing and, thereafter, tested for plasma hemoglobin concentration. The results are shown in Table 2.

Control 6

A CRC samples was placed in a stoppered polypropylene tube, left standing, and thereafter tested for plasma hemoglobin concentration. The results are shown in Table 2.

Examples 3 and 4 and Controls 4 thorugh 6 are experiments which were carried out on one and the same CRC.

Table 2

| | Reagent | Final concent-ration (mM) | Hemoglobin concentration in plasma (Mg/ml) |
|---|---|---|---|
| Example 3 | Isopropyl isotridecyl maleate | 4.0<br>0.4 | 95<br>90 |
| Example 4 | Isopropyl decyl maleate | 4.0<br>0.4 | 99<br>95 |
| Control 4 | Di-2-ethylhexyl phthalate | 0.5 | 92 |
| Control 5 | Diisopropyl maleate | 4.0<br>0.4 | 160<br>135 |
| Control 6 | - | - | 135 |

Example 5

Glycerine-1-butyl-3-isostearyl ether was added to a 2000 µg/ml polyoxyethylene monooleate (regent-grade product of Wako Junyaku K.K. marketed under trademark designation of "Tween 80")/ physiological saline mixed solution so as to be contained therein in a final concentration of 4 mM. A 0.2ml aliquot of the resultant emulsion was added to CRC adjusted in advance to a hematocrit value of about 70% so as to be contained therein in a final concentration indicated in Table 3. The resultant liquid was placed in a stoppered polypropylene tube and left standing at 4°C for 4 weeks. Thereafter, it was tested for plasma hemoglobin concentration by following the procedure of Example 1. The results are shown in Table 3.

Example 6

An emulsion was prepared by following the procedure of Example 5, except that glycerine-1,3-dioctyl ether was used in place of glycerine- 1-butyl-3-isostearyl

ether. A CRC sample incorporating the resultant emulsion was left standing and, thereafter, tested for plasma hemoglobin concentration. The results are shown in Table 3.

Controls 7 and 8

Emulsions were prepared by following the procedure of Example 5, except that di-2-ethylhexyl phthalate (Control 7) and glycerine monooctyl ether, an ether compound deviating from the scope of the present invention, (Control 8) were respectively used in place of glycerine-1-butyl-3-isostearyl ether. CRC samples incorporating the emulsions were left standing and, thereafter, tested for plasma hemoglobin concentration. The results are shown in Table 3.

Control 9

A CRC sample incorporating therein a 2000μg/ml polyoxyethylene monooleate (reagent-grade product of Wako Junyaku K.K. marketed under trademark designation of "Tween 80")/physiological saline mixed solution alone was left standing and, thereafter, tested for plasma hemoglobin concentration. The results are shown in Table 3.

Examples 5 and 6 and Controls 7 through 9 are experiments which were carried out on one and the same CRC.

Table 3

| | Reagent | Final concent-ration (mM) | Hemoglobin concentration in plasma (mg/ml) |
|---|---|---|---|
| Example 5 | Glycerine-1-butyl-3-isostearyl ether | 4.0<br>0.4 | 93<br>56 |
| Example 6 | Glycerine-1,3-dioctyl ether | 4.0<br>0.4 | 10000<br>78 |
| Control 7 | Di-2-ethylhexyl phthalate | 0.5 | 62 |
| Control 8 | Glycerine monooctyl ether | 4.0<br>0.4 | 20000<br>251 |
| Control 9 | - | - | 150 |

Example 7

Glycerine-1-butyl-isostearyl ether was added to CRC adjusted in advance to a hematocrit value of about 70% so as to be contained therein in a final concentration indicated in Table 4 and stirred gently. The resultant liquid was placed in a stoppered polypropylene tube and left standing at 4°C for 4 weeks, while subjecting to gentle stirring per a week. Thereafter, it was tested for plasma hemoglobin concentration by following the procedure of Example 1. The results are shown in Table 4.

Example 8

A CRC sample was prepared by following the procedure of Example 7, except that glycerine-1,3-dioctyl ether was used in place of glycerine-1-butyl 3-isostearyl ether. The resultant liquid was left standing and, thereafter, tested for plasma hemoglobin concentration. The results are shown in Table 4.

Controls 10 and 11

Emulsions were prepared by following the procedure of Example 7, excepting di-2-ethylhexyl phthalate (Control 10) and glycerine monooctyl ether, an ether compound deviating from the scope of the present invention, (Control

11) were respectively used in the place of glycerine-1-butyl-3-isostearyl ether. CRC samples incorporating the emulsions were left standing and, thereafter, tested for plasma hemoglobin concetnration. The results are shown in Table 4.

Control 12

A CRC sample was placed is a stoppered polypropylene tube, left standing and, thereafter, tested for plasma hemoglobin concentration by following the procedure of Example 7. The results are shown in Table 4.

Examples 7 and 8 and Controls 10 through 12 are experiments which were carried out on one and the same CRC.

Table 4

| | Reagent | Final concent-ration (mM) | Hemoglobin concentration in plasma (mg/ml) |
|---|---|---|---|
| Example 7 | Glycerine-1-butyl-3-isostearyl ether | 4.0<br>0.4 | 124<br>103 |
| Example 8 | Glycerine-1,3-dioctyl ether | 4.0<br>0.4 | 18000<br>110 |
| Control 10 | Di-2-ethylhexyl phthalate | 0.5 | 101 |
| Control 11 | Glycerine monooctyl ether | 4.0<br>0.4 | 24000<br>540 |
| Control 12 | - | - | 138 |

Example 9

Isopropyl isolaurate was dispersed in a 2000 μg/ml poloxyethylene monooleate (reagent-grade product of Wako Junyaku K.K. marketed under trademark designation of "Tween 80")/physiological saline mixed solution so as to be contained therein in a concentration of 4 mM. A 0.2-ml aliquot of the resultant emulsion was added to CRC adjusted in advance to a hematocrit value of about 70% so as to be

contained therein in a final concentration indicated in Table 5. The resultant liquid was placed in a stoppered polypropylene tube and left standing at 4°C for 4 weeks. Thereafter, it was tested for plasma hemoglobin concentration by following the procedure of Example 1. The results are shown in Table 5.

Example 10

An emulsion was prepared by following the procedure of Example 9, except that 2-ethylhexyl isostearate was used in place of isopropyl isolaurate. A CRC sample incorporating the resultant emulsion was left standing and, thereafter, tested for plasma hemoglobin concentration. The results are shown in Table 5.

Controls 13 and 14

Emulsions were prepared by following the procedrue of Example 9, except that di-2-ethylhexyl phthalate (Control 13) and ethyl 2-ethylhexanoate, a monocarboxylic ester compound deviating from the scope of the present invention, (Control 14) respectively in place of isopropyl isolaurate. CRC samples incorporating these emulsions were left standing and, thereafter, tested for plasma hemoglobin concentration. The results are shonwn in Table 5.

Control 15

A CRC sample incorporating therein a 2000μg/ml polyoxyethylene monooleate (reagent-grade product of Wako Junyaku K.K. marketed under trademark designation of "Tween 80")/physiological saline mixed solution alone was left standing and, thereafter, tested for plasma hemoglobin concentration. The results are shown in Table 5.

Examples 9 and 10 and Controls 13 through 15 are experiments which were carried out on one and the same CRC.

Table 5

| | Reagent | Final concentration (mM) | Hemoglobin concentration in plasma (mg/ml) |
|---|---|---|---|
| Example 9 | Isopropyl isolaurate | 4.0<br>0.4 | 105<br>37 |
| Example 10 | 2-ethylhexyl isostearate | 4.0<br>0.4 | 40<br>91 |
| Control 13 | Di-2-ethylhexyl phthalate | 0.5 | 35 |
| Control 14 | Ethyl-2-ethylhexanoate | 4.0<br>0.4 | 255<br>75 |
| Control 15 | - | - | 87 |

Example 11

Isopropyl isolaurate was added to CRC adjusted in advance to a hematocrit value of about 70% so as to be contained therein in a final concentration indicated in Table 4 and stirred gently. The resultant liquid was place in a stoppered polypropylene tube and left standing at 4°C for 4 weeks, while subjecting to gentle stirring par a week. Thereafter, it was tested for plasma hemoglobin concentration by following the procedure of Example 1. The results are shown in Table 6.

Example 12

An emulsion was prepared by following the procedrue of Example 11, except that 2-ethylhexyl isostearate was used in place of isopropyl isolaurate. A CRC sample incorporating the resultant emulsion was left standing and, thereafter, tested for plasma hemoglobin concentration. The results are shown in Table 6.

Controls 16 and 17

Emulsions were prepared by following the procedure of Example 11, except that di-2-ethylhexyl phthalate (Control 16) and ethyl-2-ethylhexanoate, a monocarboxylic ester compound deviating from the scope of the present

invention, (Control 17) were respectively used in place of isopropyl isolaurate. CRC samples incorporating the resultant emulsions therein were left standing and, thereafter, tested for plasma hemoglobin concentraiton. The results are shown in Table 6.

Control 18

CRC was placed in a stoppered polypropylene tube. It was left standing and, thereafter, tested for plasma hemoglobin concentration by following the procedure of Example 11. The results are shown in Table 6.

Examples 11 and 12 and Controls 15 through 18 are experiments which were carried out on one and the same CRC.

Table 6

| | Reagent | Final concent-ration (mM) | Hemoglobin concentration in plasma (mg/ml) |
|---|---|---|---|
| Example 11 | Isopropyl isolaurate | 4.0<br>0.4 | 110<br>93 |
| Example 12 | 2-ethylhexyl isostearate | 4.0<br>0.4 | 95<br>100 |
| Control 16 | Di-2-ethylhexyl phthalate | 0.5 | 87 |
| Control 17 | Ethyl-2-ethylhexanoate | 4.0<br>0.4 | 202<br>124 |
| Control 18 | - | - | 120 |

It is clearly noted from the results given in Tables 1 through 6 that the hemolysis depressants according to the present invention (Examples 1 through 12) possessed a hemolysis depressing capacity equal to that of di-2-ethylhexyl phthalate (Controls 1, 4, 7, 10, 13, and 16), a compound heretofore known to possess a hemolysis depressing ability, whereas carboxylic ester compounds (Controls 2 and 5), ether compounds (Controls 8 and 11), and

monocarboxylic ester compounds (Controls 14 and 17) which deviate from the scope of the present invention possessed virtually no ability to prevent hemolysis or conversely caused hemolysis.

Examples 13 through 15 and Controls 19 and 20

A 40 mM methanol solution of isopropyl isotridecyl maleate (Example 13), a 40 mM methanol solution of glycerine-1-butyl-3-isostearyl ether (Example 14), a 40 mM methanol solution of isopropyl isolaurate (Example 15), a 40 mM methanol solution of di-2-ethylhexyl phthalate (Control 19) and methanol containing no added compound (Control 20) were severally added to a human platelet rich plasma (count of platelets about 300,000/$mm^3$) in a fixed amount of 1/100, left standing at room temperature for 2 hours, and tested for platelet aggregation by the use of an instrument (product of Kyoto Daiichikagaku K.K. marketed under "Aggrecoder").

The percentage of transmittance of light through the solution of platelet rich plasma containing a given sample to the maximum transmittance of light through the same solution of platelet rich plasma not containing the sample (blank) was reported as the ratio of aggregation of the sample. As aggregation inducers, 5 μM ADP (adenosine diphosphoric acid) and 5 μg/ml collagen were used. The results are shown in Table 7.

Table 7

| | Reagent | Ratio of platelet aggregation (%) Aggregation inducer 5 μM ADP | 5 μg/ml collagen |
|---|---|---|---|
| Example 13 | Isopropyl isotridecyl maleate | 94.3 | 94.5 |
| Example 14 | Glycerine-1-butyl-3-isostearyl ether | 101.0 | 103.1 |
| Example 15 | Isopropyl isolaurate | 99.3 | 101.6 |
| Control 19 | Di-2-ethylhexyl phthalate | 92.1 | 89.6 |
| Control 20 | Methanol only | 100 | 97.9 |
| Blank | - | 100 | 100 |

It is clearly noted from Table 7 that di-2-ethylhexyl phthalate (Control 19) produced a discernible effect of curbing the recovery of the aggregating ability of platelets. This fact suggests the possibility that di-2-ethylhexyl phthalate, on entering the human system, will impair the function of platelets. It, therefore, implied that incorporatioin of this compound as a hemolysis depressant in blood is not proper. In contrast, the hemolysis depressants according to the present invention (Examples 13 through 15) discernibnly showed any such curbing effect as mentioned above and proved themseleves to be substances of high physiological safety. Thus, the hemolysis depressants according to the present invention are concluded to have no appreciable effect on platelets.

Examples 16 through 18

A varying flexible vinyl chloride resin composition indicated in Table 8 was kneaded with a roll at 150°C for 5 minutes and subsequently molded in the form of sheet 0.4 mm in thickness with an extruder. Miniature blood

bags having an inner volume of 20 ml were produced by supersposing two such sheets one on top of the other and high-frequency sealing pertinent portions of the superposed sheets. A human CPD added CRC adjusted in advnace to a hematocrit value of about 70% was dispensed in a fixed amount of about 20 ml among the bags and left standing at 4°C for 4 weeks. At the end of the standing, the CRC samples in the bags were tested for plasma hemoglobin concentration by following the procedure of Example 1. The test of the CRC resulting from the standing for exude concentration therein was carried out by taking a sample 0.5 ml in volume from the CRC, stirring the sample with 5 ml of isopropyl alcohol and 5 ml of diethyl ether, centrifuging the resultant mixture at 1500 x g for 10 minutes, evaporating the supernatant to dryness, dissolving the dry residue of the evaporation in 1 ml of acetonitrile, and analyzing the resultant solution by high-performance liquid chromatography. The results are shown in Table 9.

Controls 21 through 23

Similar miniature blood bags to those of Examples 16 through 18 were produced by the same procedure, except that varying flexible vinyl chloride resin compositions indicated in Table 8 were used instead. CRC samples placed in these miniature blood bags were left standing and, thereafter, tested for change of plasma hemoglobin concentration and for exude concentration. The results are shown in Table 9.

Examples 16 through 18 and Controls 21 through 23 are experiments which were carried out on one and the same CRC.

Table 8

| | Composition (parts by weight) | | | | | |
|---|---|---|---|---|---|---|
| | Example 16 | Example 17 | Example 18 | Control 21 | Control 22 | Control 23 |
| Polyvinyl chloride (P = 1300) | 100 | 100 | 100 | 100 | 100 | 100 |
| Di-2-ethylhexyl phthalate (DOP) | – | – | – | – | 50 | – |
| Dinormal decyl phthalate (DnDP) | 30 | 30 | 30 | 30 | – | 50 |
| Isopropyl decyl maleate | 20 | – | – | – | – | – |
| Isopropyl tetradecyl maleate | – | 20 | – | – | – | – |
| Isopropyl decyl itaconate | – | – | 20 | – | – | – |
| Di-2-ethylhexyl maleate | – | – | – | 20 | – | – |
| Epoxidized soy bean oil | 10 | 10 | 10 | 10 | 10 | 10 |
| Ca/Zn type stabilizer | 0.1 | 0.1 | 0.1 | 0.1 | 0.1 | 0.1 |

Table 9

| | Example 16 | Example 17 | Example 18 | Control 21 | Control 22 | Control 23 |
|---|---|---|---|---|---|---|
| Plasma hemoglobin concentration (mg/dl) | 65 | 70 | 69 | 66 | 45 | 133 |
| Exuatde concentration in CRC ( μg/ml) | | | | | | |
| DOP | – | – | – | – | 180 | – |
| DnDP | 3.1 | 3.3 | 3.2 | 3.5 | – | 5.5 |
| Isopropyl decyl maleate | 105 | – | – | – | – | – |
| Isopropyl tetradecyl maleate | – | 110 | – | – | – | – |
| Isopropyl decyl itaconate | – | – | 115 | – | – | – |
| Di-2-ethylhexyl maleate | – | – | – | 108 | – | – |

It is clearly noted from the results given in Table 9 that in the CRC samples using the blood bags of the flexible vinyl chloride resin compositions according to the present invention (Examples 16 through 18), hemolysis was curbed to a greater extent than in the CRC sample using DnDP as a plasticizer and entailing virtually no exudation of DnDP in blood (Control 23) and nearly to the same extent as in the CRC sample using DOP as a plasticizer (Control 22).

Examples 19 through 21 and Controls 24 through 26

To determine the effects brought about on platelets by the compounds used in Examples 16 through 18, the test for recovery of function of platelets was carried out by the following procedure.

First, a solution of 20,000 ppm of isopropyl decyl maleate in methanol (Example 19), a solution of 20,000 ppm of isopropyl tetradecyl maleate in methanol (Example 20), a solution of 20,000 ppm of isopropyl decyl itaconate in methanol (Example 21), a solution of 20,000 ppm of di-2-ethylhexyl maleate in methanol (Control 24), a solution of 20,000 ppm of di-2-ethylhexyl phthalate in methanol (Control 25), a solution of 20,000 ppm of dinormal decyl phthalate in methanol (Control 26), and methanol containing no added compound (blank) were prepared. These solutions were severally added to human platelet poor plasma in a fixed amount of 1/100. The samples, 2 ml in volume of the resultant platelet poor plasma liquids were severally mixed with 1 ml of a human platelet rich plasma and the Tyrode solution (containing 4 x $10^{-3}$ unit of 1 μM $PGE_1$ per ml and 3.5 mg of apyrase per ml of BSA) and were then incubated at 37°C for 90 minutes. Then, the platelets in the incubated samples were washed [Legrand et al., Eur. J. Biochem. 142,465(1984)], resuspended in a Tyrode incorporating 2 μg proteins of apyrase per ml/BSA solution (a Tyrode solution, pH 7.35, containing 0.2mM of $Cacl_2$, 1mM of $MgCl_2$ , 5 mM of HEPES, and 3.5 mg/ml BSA), and tested for maximum agregation ratio with respect to 50 μM of ADP and 10μg of collagen per

ml in the presence of 0.2 mg of fibrinogen by the use of an instrument (produced by Kyoto Daiichikagaku K.K. and marketed under trademark designation of "Aggrecoder"). The results are shown in Table 10.

Table 10

| | Additive | Maximum ratio of aggregation (%) ADP 50 µM | Collagen 10 µg/ml |
|---|---|---|---|
| Example 19 | Isopropyl decyl maleate | 49.3 | 71.6 |
| Example 20 | Isopropyl tetradecyl maleate | 40.2 | 70.1 |
| Example 21 | Isopropyl decyl itaconate | 39.5 | 69.9 |
| Control 24 | Di-2-ethylhexyl maleate | 30.5 | 60.1 |
| Control 25 | Di-2-ethylhexyl phthalate | 21.2 | 57.9 |
| Control 26 | Dinormal decyl phthalate | 39.0 | 70.5 |
| Blank | - | 41.6 | 72.4 |

The results given in Table 10, similarly to the results given in Table 7, indicate that the hemolysis depressants according to this invention showed no discernible action to curb the recovery of ability of platelet aggregation.

Example 22

A flexible vinyl chloride resin composition indicated in Table 11 was kneaded with a roll at 155°C for 5 minutes and then molded in the form of sheet 0.4 mm in thickness by the use of a press molding machine. Miniature blood bags having an inner volume of 20 ml were produced by superposing two such sheets one on top of the other and high-frequency sealing pertinent portions of the superposed sheets. A human CPD added CRC adjusted in advance to a hematocrit value of about 70% was dispensed in a fixed volume of about 20 ml among the blood bags and then left standing at 4°C for 4 weeks. Thereafter, the sample liquids

were tested for change of plasma hemoglobin concentration and exude concentration in CRC by following the procedure of Examples 16 through 18. The results are shown in Table 12.

Controls 27 thorugh 29

Similar miniature blood bags to those of Example 22 were produced by using varying flexible vinyl chloride resin compositions indicated in Table 11. The CRC samples consequently obtained were tested for change of plasma homoglobin concentration and exude concentration. The results are shown in Table 12.

Example 22 and Controls 27 through 29 are experiments which were carried out on one and the same CRC.

Table 11

| | Composition (parts by weight) | | | |
|---|---|---|---|---|
| | Example 22 | Control 27 | Control 28 | Control 29 |
| Polyvinyl chloride (p=1300) | 100 | 100 | 100 | 100 |
| Di-2-ethylhexyl phthalate (DOP) | - | 50 | - | - |
| Dinormal decyl phthalate (DnDP) | 30 | - | 50 | 30 |
| Glycerine-1-butyl-3-isostearyl ether | 20 | - | - | - |
| Glycerine-1-methyl-3-octyl ether | - | - | - | 20 |
| Epoxidized soy bean oil | 10 | 10 | 10 | 10 |
| Ca/Zn type stabilizer | 0.1 | 0.1 | 0.1 | 0.1 |

Table 12

| | Example 22 | Control 27 | Control 28 | Control 29 |
|---|---|---|---|---|
| Plasma hemoglobin concentration (mg/dl) | 60 | 46 | 89 | 139 |
| Exude concentraion in CRC (µg/ml) | | | | |
| DOP | - | 175 | - | - |
| DnDP | 3.5 | - | 4.2 | 3.6 |
| Glycerine-1-butyl-3-isostearyl ether | 120 | - | - | - |
| Glycerine-1-methyl-3-octyl ether | - | - | - | 139 |

It is clearly noted from the results given in Table 12 that in the CRC liquid contained in the miniature blood bag of the flexible vinyl chloride resin composition according to the present invention (Example 22), hemolysis was curbed to a greater extent than in the CRC liquid using DnDP as a plasticizer and entailed virtually no exudation in the blood and nearly to the same extent as in the CRC liquid using DOP (Control 27). In contrast, the degree of hemolysis was conversely increased when a flexible vinyl chloride resin composition incorporating therein glycerine-1-3-octyl ether differing in length of hydrocarbon chain from the ether compound contemplated for incorporation of the flexible vinyl chloride resin composition of the present ivention (Control 29).

Examples 23 through 25

A varying flexible vinyl chloride resin composition indicated in Table 13 was kneaded with a roll at 150°C for 5 minutes and then molded in the form of sheet 0.4 mm in thickness by the use of a press molding machine. Miniature blood bags having an inner volume of 20 ml were produced by superposing two such sheet one on top of the

other and high-frequency sealing pertinent portions of the superposed sheets. A human CPD added CRC adjusted in advance to a hematocrit value of about 70% was dispensed in a fixed amount of about 20 ml among the miniatrue blood bags and left standing at 4°C for 4 weeks. Then, the resultant CRC liquids were tested for change of plasma hemoglobin concentration and exudate concentration in CRC by following the procedure of Examples 16 through 18. The results are shown in Table 14.

Controls 30 through 32

Similar miniature blood bags to those of Example 23 were produced from a varying flexible vinyl chloride resin composition indicated in Table 13. CRC samples contained in the miniature blood bags were left standing and, thereafter, tested for change of plasma hemoglobin concentration and exude concentration. The results are shown in Table 14.

Examples 23 through 25 and Controls 30 through 32 are experiments which were carried out on one and the same CRC.

It is clearly noted from the results given in Table 14 that in the CRC liquids contained in the miniature blood bags using flexible vinyl chloride resin compositions according to the present invention (Examples 23 through 25), the hemolysis was curbed to a greater extent than in the CRC in the blood bags using DnDP as a plasticizer and entailed virtually no exudation in blood (Control 31) and nearly to the same extent as in the CRC in the blood bags using DOP as a plasticizer.

Table 13

| | Composition (parts by weight) | | | | | |
|---|---|---|---|---|---|---|
| | Example 23 | Example 24 | Example 25 | Control 30 | Control 31 | Control 32 |
| Polyvinyl chloride (P = 1300) | 100 | 100 | 100 | 100 | 100 | 100 |
| Di-2-ethylhexyl phthalate (DOP) | - | - | - | 50 | - | - |
| Dinormal decyl phthalate (DnDP) | 30 | 30 | 30 | - | 50 | 30 |
| Isopropyl isolaurate | 20 | - | - | - | - | - |
| 2-ethylhexyl isostearate | - | 20 | - | - | - | - |
| 2-ethylhexyl 2-ethylhexanoate | - | - | 20 | - | - | - |
| Ethyl-2-ethylhexanoate | - | - | - | - | - | 20 |
| Epoxidized soy bean oil | 10 | 10 | 10 | 10 | 10 | 10 |
| Ca/Zn type stabilizer | 0.1 | 0.1 | 0.1 | 0.1 | 0.1 | 0.1 |

Table 14

| | Example 23 | Example 24 | Example 25 | Control 30 | Control 31 | Control 32 |
|---|---|---|---|---|---|---|
| Plasma hemoglobin concentration (mg/dl) | 65 | 69 | 65 | 48 | 95 | 145 |
| Exudate concentration in CRC (μg/ml) | | | | | | |
| DOP | - | - | - | 210 | - | - |
| DnDP | 3.7 | 3.3 | 3.5 | - | 4.0 | 3.2 |
| Isopropyl isolaurate | 111 | - | - | - | - | - |
| 2-ethylhexyl isostearate | - | 125 | - | - | - | - |
| 2-ethylhexyl-2-ethylhexanoate | - | - | 120 | - | - | - |
| Ethyl-2-ethylhexanoate | - | - | - | - | - | 124 |

Examples 26 and 27 and Control 33

To determine the effects exerted on platelets by 2-ethylhexyl isostearate used in Example 24 and 2-ethylhexyl 2-ethylhexanoate used in Example 25, a test for the recovery of function of platelets was carried out by following the procedure of Examples 19 through 21. First, a solution of 20,000 ppm of 2-ethylhexyl isostearate in methanol (Example 26), a solution of 20,000 ppm of 2-ethylhexyl 2-ethylhexanoate in methanol (Example 27), a solution of 20,000 ppm of di-2-ethylhexyl phthalate in methanol (Control 33), and methanol containing no added compound (blank) were prepared. The procedure of Examples 19 through 21 was repeated on these solutions to determined maximum capacity for aggregation with respect to 50μM ADP and 10μg/ml collagen in the presence of 0.2 mg of fibrinogen per ml. The results are shown in Table 15.

Table 15

| | Additive | Maximum ratio of aggregation (%) | |
|---|---|---|---|
| | | ADP 50 μM | Collagen 10 μg/ml |
| Example 26 | 2-ethylhexyl isostearate | 88.2 | 92.0 |
| Example 27 | 2-ethylhexyl 2-ethylhexanoate | 89.8 | 93.3 |
| Control 33 | Di-2-ethylhexyl phthalate | 83.0 | 89.2 |
| Blank | - | 90.1 | 92.7 |

The results shown in Table 15, similarly to those of Table 7, indicate that the hemolysis depressants according to the present invention (Examples 26 and 27) produced no discernible action to curb recovery of the ability of platelet aggregation.

Example 28

Pellets were produced from a composition having 20 parts by weight of isopropyl decyl maleate incorporated in 100 parts by weight of ethylene-vinyl acetate copolymer (product of Mitsubishi Petrochemical Co., Ltd. and marketed under trademark designation of "Yukalon EVA") by the use of a biaxially extruding machine provided with a vent. Sheets were produced from the pellets by extrusion molding. Miniature blood bags having an inner volume of 20 ml were prepared by superposing two such sheets one on top of the other and high-frequency sealing pertinent portions of the superposed sheets. A human CPD added CRC adjusted in advance to a hematocrit value of about 70% was dispensed in a fixed volume of about 20 ml among the miniature blood bags and left standing at 4°C for 4 weeks. At the end of the standing, the CRCs were tested for plasma hemoglobin concentration by following the procedure of Example 1. The results are shown in Table 16.

Example 29

Miniature blood bags were produced by following the procedure of Example 28, except that a composition having 20 parts by weight of isopropyl tetradecyl maleate incorporated in 100 parts by weight of ethylene-vinyl acetate copolymer (product of Mitsubishi Petrochemical Co., Ltd. and marketed under trademark designation of "Yukalon EVA") was used instead. A CRC sample contained in these miniature blood bags was similarly treated and tested for change of plasma hemoglobin concentration. The results are shown in Table 16.

Control 34

For comparison, miniature blood bags were produced by following the procedure of Example 28, excepting ethylene-vinyl acetate copolymer (product of Mitsubishi Petrochemical Co., Ltd. and marketed under trademark designation of "Yukalon EVA") alone was used instead. A CRC sample contained in these miniature blood bags was similarly treated and tested for change of plasma hemoglobin concentration. The results are shown in Table 16.

Example 30

Miniature blood bags were produced by following the procedure of Example 28, except that polyurethane (produced by Dai-Nippon Ink & Chemicals, Inc. and marketed under trademark designation of "Pandex") was used in the place of ethylene-vinyl acetate copolymer. A CRC sample contained in these miniature bags was similarly treated and tested for change of plasma hemoglobin concentration. The results are shown in Table 16.

Example 31

Miniature blood bags were produced by following the procedure of Example 29, except that polyurethane (produced by Dai-Nippon Ink & Chemicals, Inc. and marketed under trademark designation of "Pandex") was used in place of ethylene-vinyl acetate copolymer. A CRC sample contained in these miniature bags was similarly treated and tested for change of plasma hemoglobin concentration. The results are shown in Table 16.

Control 35

For comparison, miniature blood bags were produced by following the procedure of Example 30, except that polyurethane (produced by Dai-Nippon Ink & Chemicals Inc. and marketed under trademark designation of "Pandex") was alone. A CRC sample contained in these miniature blood bags was similarly treated and tested for change of plasma hemoglobin concentration. The results are shown in Table 16.

Examples 28 through 31 and Controls 34 and 35 are experiments which were carried out on one and the same CRC.

Table 16

| | Flexible resin | Additive | Plasma Hemoglobin Concentration (mg/dℓ) |
|---|---|---|---|
| Example 28 | Ethylene-vinyl acetate copolymer | Isopropyl decyl maleate | 6.5 |
| Example 29 | Ethylene-vinyl acetate copolymer | Isopropyl tetradecyl maleate | 72 |
| Control 34 | Ethylene-vinyl acetate copolymer | —— | 140 |
| Example 30 | Polyurethane | Isopropyl decyl maleate | 70 |
| Example 31 | Polyurethane | Isopropyl tetradecyl maleate | 74 |
| Control 35 | Polyurethane | —— | 135 |

Example 32

Pellets were produced from a composition having 20 parts by weight of glycerine-1-butyl-3-isostearyl ether incorporated in 100 parts by weight of ethylene-vinyl acetate copolymer (product of Mitsubishi Petrochemical Co., Ltd. and marketed under trademark designation of "Yukalon EVA") by the use of a biaxially extruding machine provided with vents. Sheets were produced from the pellets by extrusion molding. Miniature blood bags having an inner volume of 20 ml were produced by superposing two such sheets one on top of the other and high-frequency sealing pertinent portions of the superposed sheets. A human CPD added CRC adjusted in advance to a hematocrit value of about 70% was dispensed in a fixed volume of about 20 ml among these miniatrue blood bags and left standing at 4°C for 3 weeks. After the standing, the CRC liquids were tested for change of plasma hemoglobin concentration by following the procedure of Example 1. The results are shown in Table 17.

Example 33

Miniature blood bags were produced by following the procedure of Example 32, except that a composition having 20 parts by weight of glycerine-1-octyl-3-isostearyl ether incorporated in 100 parts by weight of ethylene-vinyl acetate copolymer (product of Mitsubishi Petrochemical Co., Ltd. and marketed under trademark designation of "Yukalon EVA") was used instead. A CRC sample contained in these miniature blood bags was similarly treated and tested for change of plasma hemoglobin concentration. The results are shown in Table 17.

Control 36

For comparison, miniature blood bags were prepared by following the procedure of Example 28, except that ethylene-vinyl acetate copolymer (product of Mitsubishi Petrochemical Co., Ltd. and marketed under trademark designation of "Yukalon EVA") was used alone instead. A CRC

sample contained in these miniature blood bags was similarly treated and tested for change of plasma hemoglobin concentration. The results are shown in Table 17.

Example 34

Miniature blood bags were produced by following the procedure of Example 32, except that polyurethane (produced by Dai-Nippon Ink & Chemicals, Inc. and marketed under trademark designation of "Pandex") was used in place of ethylene-vinyl acetate copolymer. CRC contained in these miniature bags was similarly treated and tested for change of plasma hemoglobin concentration. The results are shown in Table 17.

Example 35

Miniature blood bags were produced by following the procedure of Example 32, except that polyurethane (produced by Dai-Nippon Ink & Chemicals, Inc. and marketed under trademark designation of "Pandex") was used in place of ethylene-vinyl acetate copolymer. CRC contained in these miniature bags was similarly treated and tested for change of plasma hemoglobin concentration. The results are shown in Table 17.

Control 37

For comparison, miniature blood bags were produced by following the procedure of Examaple 34, except that polyurethane (produced by Dai-Nippon Ink & Chemicals, Inc. and marketed under trademark designation of "Pandex") alone was used instead. CRC contained in the miniature blood bags was similarly treated and tested for change of plasma hemoglobin concentration. The results are shown in Table 17.

Exmaple 32 through 35 and Controls 36 and 37 are experiments which were carried out on one and the same CRC.

Table 17

| | Flexible resin | Additive | Plasma Hemoglobin Concentration (mg/dℓ) |
|---|---|---|---|
| Example 32 | Ethylene-vinyl acetate copolymer | Glycerine-1-butyl-3-isostearyl ether | 39 |
| Example 33 | Ethylene-vinyl acetate copolymer | Glycerine-1-octyl-3-isostearyl ether | 42 |
| Control 36 | Ethylene-vinyl acetate copolymer | —— | 100 |
| Example 34 | Polyurethane | Glycerine-1-butyl-3-isostearyl ether | 42 |
| Example 35 | Polyurethane | Glycerine-1-octyl-3-isostearyl ether | 42 |
| Control 37 | Polyurethane | —— | 105 |

Example 36

Pellets were produced from a composition having 20 parts by weight of isopropyl isolaurate incorporated in 100 parts by weight of ethylene-vinyl acetate copolymer (product of Mitsubishi Petrochemical Co., Ltd. and marketed under trademark designation of "Yukalon EVA") by the use of a biaxial extruding machine provided with vents. Sheets were produced from the pellets by extrusion molding. Miniature blood bags were produced by superposing two such sheets one on top of the other and high-frequency sealing pertinent portions of the superposed sheets. A human CPD added CRC adjusted in advance to a hematocrit value of about 70% was dispensed in a fixed volume of about 20 ml among the miniature blood bags and left standing at 4°C for 3 weeks. At the end of the standing, the CRC were tested for change in plasma hemoglobin concentration by following the procedure of Example 1. The results are shown in Table 18.

Example 37

Miniature blood bags were produced by following the procedure of Example 36, except that a composition having 20 parts by weight of 2-ethylhexyl isostearate incorporated in 100 parts by weight of ethylene-vinyl acetate copolymer (product of Mitsubishi Petrochemical Co., Ltd. and marketed under trademark designation of "Yukalon EVA") was used instead. CRC contained in the miniature blood bags was similarly treated and tested for change of plasma hemoglobin concentration. The results are shown in Table 18.

Example 38

Miniature blood bags were produced by following the procedure of Example 36, except that a composition having 20 parts by weight of 2-ethylhexyl 2-ethylhexanoate incorporated in 100 parts by weight of ethylene-vinyl acetate copolymer (Product of Mitsubishi Petrochemical Co., Ltd. and marketed under trademark designation of "Yukalon EVA") was used instead. CRC contained in the miniature

blood bags was similarly treated and tested for change of plasma hemoglobin concentration. The results are shown in Table 18.

Control 38

For comparison, miniature blood bags were prepared by following the procedure of Example 36, excepting ethylene -vinyl acetate copolymer (product of Mitsubishi Petrochemical Co., Ltd. and marketed under trademark designation of "Yukalon EVA") was used alone instead. CRC sample contained in these miniature blood bags was similarly treated and tested for change of plasma hemoglobin concentration. The results are shown in Table 18.

Example 39

Miniature blood bags were produced by following the procedure of Example 36, except that polyurethane (produced by Dai-Nippon Ink & Chemicals, Inc. and marketed under trademark designation of "Pandex") was used in place of ethylene-vinyl acetate copolymer. CRC contained in these miniature blood bags was similarly treated and tested for change of plasma hemoglobin concentration. The results are shown in Table 18.

Example 40

Miniature blood bags were produced by following the procedure of Example 37, except that polyurethane (produced by Dai-Nippon Ink & Chemicals, Inc. and marketed under trademark designation of "Pandex") was used in place of ethylene-vinyl acetate copolymer. CRC contained in these miniature blood bags was similarly treated and tested for change of plasma hemoglobin concentration. The results are shown in Table 18.

Example 41

Miniature blood bags were produced by following the procedure of Example 38, except that polyurethane (produced by Dai-Nippon Ink & Chemicals, Inc. and marketed under trademark designation of "Pandex") was used in place of ethylene-vinyl acetate copolymer. CRC contained in these

miniature blood bags was similarly treated and tested for change of plasma hemoglobin concentration. The results are shown in Table 18.

Control 39

For comparison, miniature blood bas were prepared by following the procedure of Example 39, except that polyurethane (produced by Dai-Nippon Ink & Chemicals, Inc. and marketed under trademark designation of "Pandex") alone was used instead. CRC sample contained in these miniature blood bags was similarly treated and tested for change of plasma hemoglobin concentration. The results are shown in Table 18.

Examples 36 through 41 and Controls 38 and 39 are experiments which were carried out on one and the same CRC.

Table 18

| | Flexible resin | Additive | Plasma Hemoglobin Concentration (mg/dℓ) |
|---|---|---|---|
| Example 36 | Ethylene-vinyl acetate copolymer | isopropyl isolaurate | 42 |
| Example 37 | Ethylene-vinyl acetate copolymer | 2-ethylhexyl isostearate | 40 |
| Example 38 | Ethylene-vinyl acetate copolymer | 2-ethylhexyl 2-ethylhexanoate | 35 |
| Control 38 | Ethylene-vinyl acetate copolymer | —— | 95 |
| Example 39 | Polyurethane | isopropyl isolaurate | 40 |
| Example 40 | Polyurethane | 2-ethylhexyl isostearate | 38 |
| Example 41 | Polyurethane | 2-ethylhexyl 2-ethylhexanoate | 35 |
| Control 39 | Polyurethane | —— | 97 |

It is clearly noted from the results given in Tables 16 through 18 that in the CRCs contained in the miniature blood bags using the flexible resin compositions according with the present invention (Examples 28 through 41), the hemolysis was curved to a greater extent than in the CRCs contained in the miniature blood bags using flexible resin composition containing no such a compound (Controls 34 through 39).

Example 42

Pellets were produced from a composition having 3 parts by weight of isopropyl decyl maleate incorporated in 100 parts by weight of polyethylene terephthalate (produced by Nippon Unipet K.K. and marketed under trademark designation of "Unipet RT560") by the use of biaxial extruding machine provided with vents. Blood collection tubes having an inner volume of 5 ml were produced from the pellets by injection molding. An EDTA-added human blood was dispensed in a fixed volume of about 3 ml among the blood collection tubes and left standing at 4°C for 3 weeks. At the end of the standing, the blood samples in the blood collection tubes were assayed for plasma hemoglobin concentration by the TMB method [Clin. Chem., 23 4 749-(1977)]. The results are shown in Table 19.

Example 43

Blood collection tubes were produced by following the procedure of Example 42, except that isopropyl tetradecyl maleate was used in place of isopropyl decyl maleate. These blood collection tubes were subjected to the same test. The results are shown in Table 19.

Control 40

For comparison, pellets were produced from polyethylene terephthalate (produced by Nippon Unipet K.K. and marketed under trademark designation of "Unipet RT560") alone. Blood collection tubes were produced from these pellets by following the procedure of Example 42 and

subjected to the same test. The results are shown in Table 19. These results are also shown in Table 20 and Table 21 to facilitate comparison.

Example 44

Blood collection tubes were produced by following the procedrue of Example 42, escepting polystyrene (produced by Nippon Steel Chemical Co., Ltd. and marketed under trademark designation of "Estyrene G-12F") was used in the place of polyethylene terephthalate. These blood collection tubes were subjected to the same test. The results are shown in Table 19.

Example 45

Blood collection tubes were produced by following th procedure of Example 44, excepting isopropyl tetradecyl maleate was used in the place of isopropyl decyl maleate. These blood collection tubes were subjected to the same test. The results are shown in Table 19.

Control 41

For comparison, blood collection tubes were prepared by following the procedure of Example 44, excepting polystyrene (produced by Nippon Steel Chemical Co., Ltd. and marketed under trademark designation of "Estyrene G-12F") alone was used instead. These blood collection tubes were subjected to the same test. The results are shown in Table 19. These results are also shown in Table 20 and Table 21 to facilitate comparison therein.

Example 46

Blood colletion tubes were produced by following the procedure of Example 42, except that polymethyl methacrylate (produced by Kyowa Gas Chemical Industry Co., Ltd. and marketed under trademark designation of "Parapet G2) was used in place of polyethylene terephthalate. These blood collection tubes were subjected to the same test. The results are shown in Table 19.

Example 47

Blood collection tubes were produced by following the procedure of Example 46, except that isopropyl tetradecyl maleate was used in the place of isopropyl decyl maleate. These blood collection tubes were subjected to the same test. The results are shown in Table 19.

Control 42

For comparison, blood collection tubes were produced by following the procedure of Example 46, except that pellets used therefor were obtained from polymethyl methacrylate (produced by Kyowa Gas Chemical Industry Co. Ltd. and marketed under trademark designation of "Parapet G") alone. These blood collection tubes were subjected to the same test. The results are shown in Table 19. These results are also shown in Table 20 and Table 21 to facilitate comparison therein.

Example 48

Blood collection tubes were produced by following the procedure of Example 42, except that polyacrylonitrile (produced by Vestron Corp. and marketed under trademark designation of "BAREX 210") was used in the place of polyethylene terephthalate. These blood collection tubes were subjected to the same test. The results are shown in Table 19.

Example 49

Blood collection tubes were produced by following the procedure of Example 46, except that isopropyl tetradecyl maleate was used in the place of isopropyl decyl maleate. These blood collection tubes were subjected to the same test. The results are shown in Table 19.

Control 43

For comparison, blood collection tubes were produced by following the procedure of Example 48, except that pellets used therefor were obtained from polyacrylonitrile (produced by Vestron Corp. and marketed under trademark designation of "BAREX 210") alone. These

blood collection tubes were subjected to the same test. The results are shown in Table 19. These results are also shown in Table 20 and Table 21 to facilitate comparison therein.

Example 50

Pellets were produced from a composition having 3 parts by weight of glycerine-1-butyl-3-isostearyl ether incorporated in 100 parts by weight of polyethylene terephthalate (produced by Nippon Unipet K.K. and marketed under trademark designation of "Unipet RT 560") by the use of a biaxial extruding machine provided with vents. Blood collection tubes having an inner volume of 5 ml were produced from the pellets by injection molding. An EDTA-added human blood was dispensed in a fixed volume of about 3 ml among the blood collection tubes and left standing at 4°C for 3 weeks. At the end of the standing, the blood samples were tested for change of plasma hemoglobin concentration by following the procedure of Example 1. The results are shown in Table 20.

Example 51

Blood collection tubes were produced by following the procedure of Example 50, except that glycerine-1-octyl-3-isostearyl ether was used in place of glycerine-1-butyl-3-isostearyl ether. These blood collection tubes were subjected to the same test. The results are shown in Table 20.

Example 52

Blood collection tubes were produced by following the procedure of Example 50, except that polystyrene (produced by Nippon Steel Chemical Co., Ltd. and marketed under trademark designation of "Estyrene G-12F") was used in place of polyethylene terephthalate. These blood collection tubes were subjected to the same test. The results are shown in Table 20.

Example 53

Blood collection tubes were produced by following the procedure of Example 52, excepting glycerine-1-octyl-3-isostearyl ether was used in the place of glycerine-1-butyl-3-isostearyl ether. These blood collection tubes were subjected to the same test. The results are shown in Table 20.

Example 54

Blood collection tubes were produced by following the procedure of Example 50, except that polymethyl methacrylate (produced by Kyowa Gas Chemical Industry Co., Ltd.and marketed under trademark designation of "Parapet G") was used in the place of polyethylene terephthalate. These blood collection tubes were subjected to the same test. The results are shown in Table 20.

Example 55

Blood collection tubes were produced by following the procedure of Example 54, except that glycerine-1-octyl-3-isostearyl ether was used in the place of glycerine-1-butyl-3-isostearyl ether. These blood collection tubes were subjected to the same test. The results are shown in Table 20.

Example 56

Blood collection tubes were produced by following the procedure of Example 50, except that polyacrylonitrile (produced by Vestron Corp. and marketed under trademark designation of "BAREX 210") was used in place of polyethylene terephthalate. The blood collection tubes were subjected to the same test. The results are shown in Table 20.

Example 57

Blood collection tubes were produced by following the procedure of Example 56, except that glycerine-1-octyl-3-isostearyl ether was used in place of glycerine-1-butyl-3-isostearyl ether. The blood collection tubes were subjected to the same test. The results are shown in Table 20.

Example 58

Pellets were produced from a composition having 3 parts by weight of isopropyl isolaurate incorporated in 100 parts by weight of polyethylene terephthalate (produced by Nippon Unipet K.K. and marketed under trademark designation of "Unipet RT 560") by the use of a biaxial extruding machine provided with vents. Blood collection tubes having an inner volume of 5 ml were produced from the pellets by injection molding. An EDTA-added human blood was dispensed in a fixed volume of about 3 ml among the blood collection tubes and left standing at 4°C for 3 weeks. At the end of the standing, the blood samples were tested for change of plasma hemoglobin concentration by following the procedure of Example 1. The results are shown in Table 21.

Examples 59 and 60

Blood collection tubes were produced by following the procedure of Example 58, except that 2-ethylhexyl isostearate (Example 59) and 2-ethylhexyl 2-ethylhexanoate (Example 60) were respectively used in place of isopropyl isolaurate. The blood collection tubes were subjected to the same test. The results are shown in Table 21.

Example 61:

Blood collection tubes were produced by following the procedure of Example 58, except that polystyrene (produced by Nippon Steel Chemical Co., Ltd. and marketed under trademark designatin of "Estyrene G-12F") was used in place of polyethylene terephthalate. The blood collection tubes were subjected to the same test. The results are shown in Table 21.

Examples 62 and 63

Blood collection tubes were produced by following teh procedure of Example 61 except that 2-ethylhexyl isostearate (Example 62) and 2-ethylhexyl 2-ethylhexanoate (Example 63) were used respectively in place of isopropyl isolaurate. The blood collection tubes were subjected to the same test. The results are shown in Table 21.

Example 64

Blood collection tubes were produced by following the procedrue or Example 58, except that polymethyl methacrylate (produced by Kyowa Gas Chemical Industry. Co., Ltd. and marketed under trademrk designation of "Parapet G") was used in place of polyethylene terephthalate. The blood collection tubes were subjected to the same test. The results are shown in Table 21.

Examples 65 and 66

Blood collection tubes were produced by following the procedure of Example 64, except that 2-ethylhexyl isostearate (Example 65) and 2-ethylhexyl 2-ethylhexanoate (Example 66) were used respectively in place of isopropyl isolaurate. The blood collection tubes were subjected to the same test. The results are shown in Table 21.

Example 67

Blood collection tubes were produced by following the procedure of Example 58, except that polyacrylonitrile (produced by Vestron Corp. and marketed under trademark designation of "BAREX 210") was used in place of polyethylene terephthalate. The blood collection tubes were subjected to the same test. The results are shown in Table 21.

Examples 68 and 69

Blood collection tubes were produced by following the procedure of Example 67, except that 2-ethylhexyl isosterate (Example 68) and 2-ethylhexyl 2-ethylhexanoate (Example 69) were used respectively in place of isopropyl isolaurate. The blood collection tubes were subjected to the same test. The results are shown in Table 21.

Examples 42 through 69 and Controls 40 through 43 are experiments which were carried out on one and the same human blood.

Table 19

| | Rigid resin | Additive | Plusma Hemoglobin Concentration *(mg/dℓ) |
|---|---|---|---|
| Example 42 | Polyethylene terephtalate | isopropyl decyl maleate | 45 |
| Example 43 | Polyethylene terephtalate | isopropyl tetradecyl maleate | 50 |
| Control 40 | Polyethylene terephtalate | —— | 105 |
| Example 44 | Polystyrene | isopropyl decyl maleate | 33 |
| Example 45 | Polystyrene | isopropyl tetradecyl maleate | 51 |
| Control 41 | Polystyrene | —— | 110 |
| Example 46 | Polymethyl methacrylate | isopropyl decyl maleate | 40 |
| Example 47 | Polymethyl methacrylate | isopropyl decyl maleate | 53 |
| Control 42 | Polymethyl methacrylate | —— | 97 |
| Example 48 | Polyacrylonitrile | isopropyl decyl maleate | 35 |
| Example 49 | Polyacrylonitrile | isopropyl tetradecyl maleate | 58 |
| Control 43 | Polyacrylonitrile | —— | 120 |

* The indicated values of the plasma hemoglobin concentration were represented by the average of three data, respectively.

Table 20

| | Rigid resin | Additive | Plusma Hemoglobin Concentration *(mg/dℓ) |
|---|---|---|---|
| Example 50 | Polyethylene terephtalate | Glycerine-1-butyl-3-isostearyl ether | 70 |
| Example 51 | Polyethylene terephtalate | Glycerine-1-octyl-3-isostearyl ether | 80 |
| Control 40 | Polyethylene terephtalate | ——— | 105 |
| Example 52 | Polystyrene | Glycerine-1-butyl-3-isostearyl ether | 75 |
| Example 53 | Polystyrene | Glycerine-1-octyl-3-isostearyl ether | 83 |
| Control 41 | Polystyrene | ——— | 110 |
| Example 54 | Polymethyl methacrylate | Glycerine-1-butyl-3-isostearyl ether | 70 |
| Example 55 | Polymethyl methacrylate | Glycerine-1-octyl-3-isostearyl ether | 84 |
| Control 42 | Polymethyl methacrylate | ——— | 97 |
| Example 56 | Polyacrylonitrile | Glycerine-1-butyl-3-isostearyl ether | 66 |
| Example 57 | Polyacrylonitrile | Glycerine-1-octyl-3-isostearyl ether | 80 |
| Control 43 | Polyacrylonitrile | ——— | 120 |

* The indicated values of the plasma hemoglobin concentration were represented by the average of three data, respectively.

Table 21

| | Rigid resin | Additive | Plusma Hemoglobin Concentration *(mg/dℓ) |
|---|---|---|---|
| Example 58 | Polyethylene terephtalate | Isopropyl isolaurate | 68 |
| Example 59 | Polyethylene terephtalate | 2-ethylhexyl isostearate | 73 |
| Example 60 | Polyethylene terephtalate | 2-ethylhexyl 2-ethylhexanoate | 59 |
| Control 40 | Polyethylene terephtalate | ——— | 105 |
| Example 61 | Polystyrene | Isopropyl isolaurate | 70 |
| Example 62 | Polystyrene | 2-ethylhexyl isostearate | 79 |
| Example 63 | Polystyrene | 2-ethylhexyl 2-ethylhexanoate | 62 |
| Control 41 | Polystyrene | ——— | 110 |

Table 21 (continued)

| | | | |
|---|---|---|---|
| Example 64 | Polymethyl methacrylate | Isopropyl isolaurate | 65 |
| Example 65 | Polymethyl methacrylate | 2-ethylhexyl isostearate | 72 |
| Example 66 | Polymethyl methacrylate | 2-ethylhexyl 2-ethylhexanoate | 70 |
| Control 42 | Polymethyl methacrylate | —— | 97 |
| Example 67 | Polyacrylonitrile | Isopropyl isolaurate | 65 |
| Example 68 | Polyacrylonitrile | 2-ethylhexyl isostearate | 78 |
| Example 69 | Polyacrylonitrile | 2-ethylhexyl 2-ethylhexanorate | 65 |
| Control 43 | Polyacrylonitrile | —— | 120 |

* The indicated values of the plasma hemoglobin concentration were represented by the average of three data, respectively.

It is clearly noted from the results given in Tables 19 through 21 that in the CRC liquids contained in the blood collection tubes using the rigid resin compositions according wtih the present invention (Examples 42 through 69), the hemolysis was curbed to a greater extent than in the CRC liquids contained in the blood collection tubes using rigid resin compositions incorporating no hemolysis depressant and experiencing no exudation in blood (Controls 40 through 43).

Example 70

An emulsion was produced by dissolving Tween 80 (reagent grade produced by Wako Junyaku K.K.) in a SAG solution (containing 140 mM of NaCl, 1.25 mM of adenine and 50 mM of glucose) so as to be contained therein in a concentration of 600 μg/ml adding 12 mM isopropyl isotridecyl maleate to the resultant solution, and stirring the resultant mixture for uniform dispersion. In a stoppered polypropylene tube, a mixture of 1.0 ml of the emulsion prepared as described above and 2.0 ml of a liquid of concentrated human red cells adjusted in advance to a hematocrit value of about 70% was left standing at 4°C for 5 weeks. The resultant liquid was tested for free hemoglobin concentration in plasma by following the procedure of Example 1. The results are shown in Table 22.

Exmaple 71

A blood preserving liquid was prepared by following the procedure of Example 70, except that the amount of isopropyl isotridecyl maleate to be added was changed to 1.2mM. The blood preserving liquid was treated and tested for change of free hemoglobin concentration in plasma. The results are shown in Table 22.

Example 72

A blood preserving liquid was prepared by following the procedure of Example 70, except that isopropyl decyl maleate was used in the place of isopropyl isotridecyl

maleate. It was similarly treated and tested for change of free hemoglobin concentration in plasma. The results are shown in Table 22.

Example 73

A blood preserving liquid was prepared by following the procedure of Example 72, except that the amount of isopropyl decyl maleate to be added was changed to 1.2 mM. This blood preserving liquid was similarly treated and tested for change of free hemoglobin concentration in plasma. The results are shown in Talbe 22.

Control 44

For comparison, a blood preserving liquid was prepared by following the procedure of Example 70, except that diisopropyl maleate, a carboxylic ester compound deviating from the scope of this invention, was used in place of isopropyl isotridecyl maleate. The blood preserving liquid was similarly treated and tested for change of free hemoglobin concentration in plasma. The results are shown in Table 22.

Control 45

A blood preserving liquid was prepared by following the procedure of Control 44, except that the amount of diisopropyl maleate to be added was changed to 1.2 mM. This blood preserving liquid was similarly treated and tested for change of free hemoglobin concentration in plasma. The results are shown in Table 22.

Control 46

For comparison, a blood preserving liquid was prepared by following the procedure of Example 70, except that di-2-ethylhexyl phthalate, a compound heretofore known as possessing an ability to curb hemolysis, was used in place of isopropyl isolaurate. The blood presserving liquid was similarly treated and tested for change of free hemoglobin concentration in plasma. The results are shown in Table 22.

Control 47

A blood preserving liquid was prepared by following the procedure of Control 46, except that the amount of di-2-ethylhexyl phthalte to be added was changed to 1.2mM. The blood preserving liquid was similarly treated and tested for change of free hemoglobin concentration in plasma. The results are shown in Table 22.

Example 70 through 73 and Controls 44 through 47 are experiments which were carried out one and the same CRC.

Table 22

| | Additive | Final Concentration of the Additive in CRC (mM) | Concentration of Free Hemoglobin in Plasma (mg/dℓ) |
|---|---|---|---|
| Example 70 | Isopropyl isotridecyl maleate | 4.0 | 58 |
| Example 71 | Isopropyl isotridecyl maleate | 0.4 | 69 |
| Example 72 | Isopropyl decyl maleate | 4.0 | 88 |
| Example 73 | Isopropyl decyl maleate | 0.4 | 92 |
| Control 44 | Diisopropyl maleate | 4.0 | 240 |
| Control 45 | Diisopropyl maleate | 0.4 | 235 |
| Control 46 | Di-2-ethylhexyl phthalate | 4.0 | 70 |
| Control 47 | Di-2-ethylhexyl phthalate | 0.4 | 96 |
| Blank | ——— | - | 214 |

Example 74

An emulsion was prepared by dissolving Tween 80 (reagent grade product of Wako Junyaku K.K.) in a SAG liquid so as to be contained therein in a concentration of 600 μg/ml, adding 12 mM of glycerine-1-butyl-3-isostearyl ether and stirring the resultant mixture for uniform dispersion. In a stoppered polypropylene tube, a mixture of 1.0 ml of the emulsion prepared as described above and 2.0 ml of CRC adjusted in advance to a hematocrit value of about 70% was left stnading at 4°C for 5 weeks. At the end of this standing, the CRC liqud was tested for change of free hemoglobin concentration in plasma by following the procedure of Example 1. The results are shown in Table 23.

Example 75

A blood preserving liquid was prepared by following the procedure of Example 74, except that the amount of glycerine-1-butyl-3-isostearyl ether to be added was changed to 1.2 mM. The blood preserving liquid was similarly treated and tested for change of free hemoglobin concentration in plasma. The results are shown in Table 23.

Example 76

A blood preserving liquid was prepared by following the procedure of Example 74, except that glycerine-1,3-dioctyl ether was used in place of glycerine-2-butyl-3-isostearyl ether. The blood preserving liquid was similarly treated and tested for change of free hemoglobin concentration in plasma. The results are shown in Table 23.

Example 77

A blood preserving liquid was prepared by following the procedure of Example 76, except that the amount of glycerine-1,3-dioctyl ether to be added was change to 1.2mM. The blood preserving liquid was similarly treated and tested for change of free hemoglobin concentration in plasma. The results are shown in Table 23.

Control 48

A blood preserving liquid was prepared by following the procedure of Example 74, except that glycerine monooctyl ether, an ether compound deviating from the scope of this invention, was used in place of glycerine-1-butyl-3-isostearyl ether. The blood preserving liquid was similarly treated and tested for change of free hemoglobin concentration in plasma. The results are shown in Table 23.

Control 49

A blood preserving liquid was prepared by following the procedure of Control 48, except that the amount of glycerine monooctyl ether to be added was changed to 1.2 mM. The blood preserving liquid was similarly treated and tested for change of free hemoglobin concentration in plasma. The results are shown in Table 23.

Control 50

For comparison, a blood preserving liquid was prepared by following the procedure of Example 74, except that di-2-ethylhexyl phthalate, a compound heretofore known as possessing an ability to curb hemolysis, was used in place of glycerine-1-butyl-3-isostearyl ether. The blood preserving liquid was similarly treated and tested for change of free hemoglobin concentration in plasma. The results are shown in Table 23.

Control 51

A blood preserving liquid was prepared by following the procedure of Control 50, except that the amount of di-2-ethylhexyl phthalate to be added was changed to 1.2mM. The blood preserving liquid was similarly treated and tested for change of free hemoglobin concentration in plasma. The results are shown in Table 23.

Examples 74 through 77 and Controls 48 through 51 are experiments which were carried out on one and the same CRC.

Table 23

| | Additive | Final Concentration of the Additive in CRC (mM) | Concentration of Free Hemoglobin in Plasma (mg/dℓ) |
|---|---|---|---|
| Example 74 | Glycerine-1-butyl-3-isostearyl ether | 4.0 | 102 |
| Example 75 | Glycerine-1-butyl-3-isostearyl ether | 0.4 | 71 |
| Example 76 | Glycerine-1,3-dioctyl ether | 4.0 | 2500 |
| Example 77 | Glycerine-1,3-dioctyl ether | 0.4 | 96 |
| Control 48 | Glycerine monooctyl ether | 4.0 | 8000 |
| Control 49 | Glycerine monooctyl ether | 0.4 | 379 |
| Control 50 | Di-2-ethylhexyl phthalate | 4.0 | 70 |
| Control 51 | Di-2-ethylhexyl phthalate | 0.4 | 83 |
| Blank | ——— | ——— | 186 |

Example 78

An emulsion was prepared by dissolving Tween 80 (reagent grade product of Wako Junyaku K.K.) in a SAG liquid so as to be contained therein in a concentration of 600 μg/ml, adding 12 mM of isopropyl isolaurate to the resultant solution, and stirring the resultant mixture for uniform dispersion. In a stoppered polypropylene tube, a mixture of 1.0 ml of the emulsion prepared as described above and 2.0 ml of CRC adjusted in advance to a hematocrit value of about 70% and then left standing at 4°C for 5 weeks. At the end of the satnding, the liquid was tested for free hemoglobin concentraiton in plasma by following the procedure of Example 1. The results are shown in Talbe 24.

Example 79

A blood preserving liquid was prepared by following the procedure of Example 78, except that the amount of isopropyl laurate to be added was changed to 1.2 mM. The blood preserving liquid was similarly treated and tested for change of hemoglobin concnetration in plasma. The results are shown in Talbe 24.

Example 80

A blood preserving liquid was prepared by following the procedure of Example 78, except that 2-ethylhexyl isostearate was used in place of isopropyl isolaurate. The blood preserving liquid was similarly treated and tested for change of free hemoglobin concentration in plasma. The results are shown in Table 24.

Example 81

A blood preserving liquid was prepared by following the procedure of Example 80, except that the amount of 2-ethylhexyl isostearate to be added was changed to 1.2 mM. The blood preserving liquid was similarly treated and tested for change of free hemoglobin concentration in plasma. The results are shown in Table 24.

Control 52

For comparison, a blood preserving liquid was prepared by following the procedure of Exampl 78, except that ethyl 2-ethylhexanoate, a monocarboxylic ester compound deviating from the scope of the present invention, was used in place of isopropyl isolaurate. This blood preserving liquid was similarly treated and tested for change of free hemoglobin concentration in plasma. The results are shown in Talbe 24.

Control 53

A blood preserving liquid was prepared by following the procedure of Control 52, except that the amount of ethyl 2-ethylhexanoate to be added was changed to 1.2 mM. The blood preserving liquid was similarly treated and tested for chagne of free hemoglobin concentation in plasma. The results are shown in Table 24.

Control 54

For comparison, a blood preserving liquid was prepared by following the procedure of Example 78, except that di-2-ethylhexyl phthalate, a compound heretofore known as possessing an ability was replaced by isopropyl isolaurate. The blood preserving liquid was similarly treated and tested for change of free hemoglobin concentration in plasma. The results are shown in Table 24.

Control 55

A blood preserving liquid was prepared by following the procedure of Control 54, except that the amount of di-2-ethylhexyl phthalate to be added was changed to 1.2 mM. The blood preserving liquid was similarly treated and tested for change of free hemoglobin concentration in plasma. The results are shown in Talbe 24

Example 78 through 81 and Controls 52 through 55 are experiments which were carried out on one and the same CRC.

Table 24

| | Additive | Final Concentration of the Additive in CRC (mM) | Concentration of Free Hemoglobin in Plasma (mg/dℓ) |
|---|---|---|---|
| Example 78 | Isopropyl isolaurate | 4.0 | 230 |
| Example 79 | Isopropyl isolaurate | 0.4 | 86 |
| Example 80 | 2-ethylhexyl isostearate | 4.0 | 97 |
| Example 81 | 2-ethylhexyl isostearate | 0.4 | 214 |
| Control 52 | Ethyl 2-ethylhexanoate | 4.0 | 722 |
| Control 53 | Ethyl 2-ethylhexyl phthalate | 0.4 | 189 |
| Control 54 | Di-2-ethylhexyl phthalate | 4.0 | 70 |
| Control 55 | Di-2-ethylhexyl phthalate | 0.4 | 85 |
| Blank | ——— | ——— | 207 |

It is clearly noted from Tables 22 through 24 that the blood preserving liquids incorporating therein the hemolysis depressants according to the present invention (Example 70 through 81) manifested an ability to curb hemolysis similarly to those incorporating di-2-ethylhexyl phthlate (Controls 46, 47, 50, 51, 54, and 55), whereas some of those incorporating therein compounds deviating from the scope of this invention showed virtually no ability to curb hemolysis and other conversely induced hemolysis.

[Industrial Applicability]

As described above, the hemolysis depressant according with the present invention produces the distinguished protective action with regard to erythrocytes, and possesses high safety. Furthermore, the hemolysis depressant is capable of being to various forms, for example, the form incorporated in a varying synthetic resin composition, and the emulsion form into a varying aqueous medium. The hemolysis depressant is enabled to prevent erythrocytes from hemolysis evenwhen it will be in these forms. Therefore, when the hemolysis depressant of this invention is added to an erythrocyte-containing liquid such as whole blood or concentrated red cells, most of the erythrocytes can be retained for a long time in the same state as immediately after blood extraction. Thus, it should be said that the present invention offers distinguished contribusion to various technical fields, including transfusionics clinical analytic as well as medicine.

CLAIMS

1. A hemolysis depressant, comprising one member or a mixture of two or more members selected from the group consisting of:

(A) a carboxylic ester compound

i) possessing at least two ester bonds,

ii) having monovalent hydrocarbon groups coupled with the ester bonds, at least two of hydrocarbon groups being chain type hydrocarbon groups differing in number of carbon atoms,

iii) at least one of said monovalent hydrocarbon groups is a hydrocarbon group of not more than 3 or not less than 13 carbon atoms, providing that all of said monovalent hydrocarbon groups are not hydrocarbon groups of not more than 3 carbon atoms or hydrocarbon groups of not less than 13 carbon atoms, and

iv) at least one of said monovalent hydrocarbon groups possesses a branched structure, and

v) possessing a molecular weight of not more than 1,000;

(B) an ether compound possessing at least one ether bond and chain type monovalent hydrocarbon groups having 3 to 20 carbon atoms coupled with the ether oxygen, the molecular weight of the ether compound being not more than 1,000; and

(C) a monocarboxylic ester compound represented by the general formula(V):

$$RCOOR' \qquad (V)$$

wherein R and R' independently stand for a chain type hydrocarbon group of not less than 3 carbon atoms, providing that the total of the carbon atoms or R and R' falls in the range of 11 to 30).

2. A hemolysis depressant according to Claim 1, wherein said carboxylic ester compound (A) possesses two ester bonds.

3. A hemolysis depressant according to Claim 1, wherein said carboxylic ester compound (A) is a dicarboxylic ester.

4. A hemolysis depressant according to Claim 1, wherein one of the monovalent hydrocarbon groups coupled with the ester bonds of said carboxylic ester compound (A) is hydrocarbon group of three carbon atoms and the other monovalent hydrocarbon groups are hydrocarbon groups of 8 to 20 carbon atoms.

5. A hemolysis depressant according to Claim 3, wherein the divalent hydrocarbon groups in the acid moiety of said dicarboxylic ester are hydrocarbon groups of 2 to 12 carbon atoms.

6. A hemolysis depressant according to Claim 3, wherein the acid moiety of said dicarboxylic ester is the residue of maleic acid, succinic acid, glutaric acid, adipic acid, itaconic acid, sebacic acid, dodecandioic acid, or oxalacetic acid.

7. A hemolysis depressant according to Claim 1, wherein said carboxylic ester compound (A) is selected from the group consisting of isopropyl 2-ethylhexyl maleate, isopropyl decyl maleate, isopropyl isotridecyl maleate, isopropyl tetradecyl maleate, and isorpopyl myristyl maleate.

8. A hemolysis depressant according to Claim 1, wherein at least one of the monovalent hydrocarbon groups coupled with the ether oxygen of said ether compound (B) possesses a branched structure.

9. A hemolysis depressant according to Claim 1, wherein said ether compound (B) possesses at least two ether bonds.

10. A hemolysis depressant according to Claim 9, wherein at least two of the monovalent hydrocarbon groups coupled with the ether oxygen possess chains different in length.

11. A hemolysis depressant according to Claim 1, wherein said ether compound (B) is glycerine diether represented by the general formula (IV') :

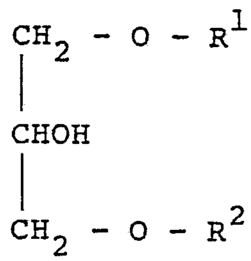

(IV')

wherein $R^1$ and $R^2$ are independently a chain type hydrocarbon group of 3 to 20 carbon atoms.

12. A hemolysis depressant according to Claim 1 wherein said glycerine diether represented by the general formula (IV') is glycerine-1-butyl-3-isostearyl ether or glycerine-1,3-bis(2-ethylhexyl) ether.

13. A hemolysis depressant according to Claim 1, wherein at least either of the substituents, R and R', in the general formula (V) of said monocarboxylic ester compound (C) possesses a branched structure.

14. A hemolysis depressant according to Claim 1, wherein the compound represented by the general formula (V) is isopropyl isolaurate, isopropyl oleate, 2-ethylhexyl isostearate, or 2-ethylhexyl 2-ethylhexanoate.

15. A flexible vinyl chloride type resin composition for medical use, characterized by comprising vinyl chloride resin, a plasticizer, and at least one hemolysis depressant selected from the group consisting of:

(A) a carboxylic ester compound

i) possessing at least two ester bonds,

ii) having monovalent hydrocarbon groups coupled with the ester bonds, at least two of hydrocarbon groups being chain type hydrocarbon groups differing in number of carbon atoms,

iii) at least one of said monovalent hydrocarbon groups is a hydrocarbon group of not more than 3 or not less than 13 carbon atoms, providing that all of said monovalent hydrocarbon groups are not hydrocarbon groups of not more than 3 carbon atoms or hydrocarbon groups of not less than 13 carbon atoms, and

iv) at least one of said monovalent hydrocarbon groups possesses a branched structure, and

v) possessing a molecular weight of not more than 1,000;

(B) an ether compound possessing at least one ether bond and chain type monovalent hydrocarbon groups having 3 to 20 carbon atoms coupled with the ether oxygen the molecular weight of the ether compound being not more than 1,000; and

(C) a monocarboxylic ester compound represented by the general formula(V):

$$RCOOR' \qquad (V)$$

wherein R and R' are independently a chain type hydrocarbon group of not less than 3 carbon atoms, providing that the total of the carbon atoms or R and R' falls in the range of 11 to 30.

16. A flexible vinyl chloride type resin composition for medical use according to Claim 15, wherein said plasticizer is incorporated in an amount in the range of 10 to 45% by weight and said hemolysis depressant in the range of 1 to 30% by weight.

17. A flexible vinyl chloride type resin composition for medical use according to Claim 15 or Claim 16, wherein said carboxylic ester compound (A) possesses two ester bonds.

18. A flexible vinyl chloride type resin composition for medical use according to Claim 15 or Claim 16, wherein said carboxylic ester compound (A) is a dicarboxylic ester.

19. A flexible vinyl chloride type resin composition for medical use according to Claim 15 or Claim 16, wherein one of the monovalent hydrocarbon groups coupled with the ester bonds of said carboxylic ester compound (A) is a hydrocarbon group of 3 carbon atoms and the other monovalent hydrocarbon groups are hydrocarbon groups of 8 to 20 carbon atoms.

20. A flexible vinyl chloride type resin composition for medical use according to Claim 18, wherein the divalent hydrocarbon groups in the acid moiety of said dicarboxylic ester are hydrocarbon groups of 2 to 12 carbon atoms.

21. A flexible vinyl chloride type resin composition for medical use according to Claim 18, wherein the acid moiety of said dicarboxylic ester is the residue of maleic acid, succinic acid, glutaric acid, adipic acid, itaconic acid, sebacic acid, dodecandioic acid, or oxalacetic acid.

22. A flexible vinyl chloride type resin composition for medical use according to Claim 15 or Claim 16, wherein said carboxylic ester compound (A) is selected from the group consisting of isopropyl 2-ethylhexyl maleate, isopropyl decyl maleate, isopropyl isotridecyl maleate, isopropyl tetradecyl maleate, and isopropyl myristyl maleate.

23. A flexible vinyl chloride type resin composition for medical use according to Claim 15 or Claim 16, wherein at least one of the monovalent hydrocarbon groups coupled with the ether oxygen of said ether compound (B) possesses a branched strucutre.

24. A flexible vinyl chloride type resin composition for medical use according to Claim 15 or Claim 16, wherein said ether compound (B) possesses at least two ether bonds.

25. A flexible vinyl chloride type resin composition for medical use according to Claim 24, wherein at least two of the monovalent hydrocarbon groups coupled with the ether oxygen possess chains different in length.

26. A flexible vinyl chloride type resin composition for medical use according to Claim 15 of Claim 16, wherein said ether compound (B) is glycerine diether represented by the general formula (IV'):

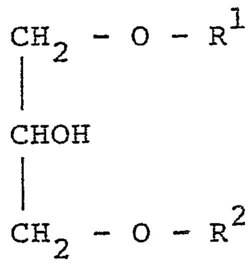

(IV')

wherein $R^1$ and $R^2$ are independently a chain type hydrocarbon group of 3 to 20 carbon atoms.

27. A flexible vinyl chloride type resin composition for medical use according to Claim 15 or Claim 16, wherein said glycerine diether represented by the general formula (IV') is glycerine-1-butyl-3-isostearyl ether or glycerine-1,3-bis(2-ethylhexyl) ether.

28. A flexible vinyl chloride type resin composition for medical use according to Claim 15 or Claim 16, wherein at least either of the substituents R and R', in the general formula (V) of said monocarboxylic ester compound (C) possessses a branched structure.

29. A flexible vinyl chloride type resin composition for medical use according to Claim 15 or Claim 16, wherein the compound represented by the general formula (V) is isopropyl isolaurate, isopropyl oleate, 2-ethylhexyl isostearate, or 2-ethylhexyl 2-ethylhexanoate.

30. A flexible vinyl chloride type resin composition for medical use according to Claim 15 or Claim 16, wherein said plasticizer possesses a low exuding property.

31. A flexible vinyl chloride type resin composition for medical use according to Claim 15 or Claim 16, wherein said plasticizer is selected from the group consisting of trialkyl trimellitates, dinormal alkyl phthalates, and tetraalkyl pyromellitates.

32. A flexible vinyl chloride type resin composition for medical use according to Claim 31, wherein said plasticizer is dinormal decyl phthalte.

33. A flexible vinyl chloride type resin composition for medical use according to Claim 31, wherein said plasticizer is trioctyl trimellitate.

34. A flexible resin composition for medical use, characterized by having at least one hemolysis depressant selected form the group consisting of:

(A) a carboxylic ester compound

i) possessing at least two ester bonds,

ii) having monovalent hydrocarbon groups coupled with the ester bonds, at least two of hydrocarbon groups being chain type hydrocarbon groups differing in number of carbon atoms,

iii) at least one of said monovalent hydrocarbon groups is a hydrocarbon group of not more than 3 or not less than 13 carbon atoms, providing that all of said monovalent hydrocarbon groups are not hydrocarbon groups of not more than 3 carbon atoms or hydrocarbon groups of not less than 13 carbon atoms, and

iv) at least one of said monovalent hydrocarbon groups possesses a branched structure, and

v) possessing a molecular weight of not more than 1,000;

(B) a ether compound possessing at least one ether bond and chain type monovalent hydrocarbon groups having 3 to 20 carbon atoms coupled with the ether oxygen, the molecular weight of the ether compound being not more than 1,000; and

(C) a monocarboxylic ester compound represented by the general formula(V):

$$RCOOR' \qquad (V)$$

wherein R and R' are independently a chain type hydrocarbon group of not less than 3 carbon atoms, providing that the total of the carbon atoms or R and R' falls in the range of 11 to 30,

incorporated in a flexible resin composition containing no plasticizer.

35. A flexible resin composition for medical use according to Claim 34, wherein said hemolysis depressant is incorporated therein in an amount in the range of 5 to 35% by weight.

36. A flexible resin composition for medical use according to Claim 34 or Claim 35, wherein said carboxylic ester compound (A) possesses two ester bonds.

37. A flexible resin composition for medical use according to Claim 34 or Claim 35, wherein said carboxylic ester compound (A) is a dicarboxylic ester.

38. A flexible resin composition for medical use according to Claim 34 or Claim 35, wherein one of the monovalent hydrocarbon groups coupled with the ester bonds of said carboxylic ester compound (A) is hydrocarbon group of three carbon atoms and the other monovalent hydrocarbon groups are hydrocarbon groups of 8 to 20 carbon atoms.

39. A flexible resin composition for medical use according to Claim 37, wherein the divalent hydrocarbon groups in th acid moiety of said dicarboxylic ester are hydrocarbon groups of 2 to 12 carbon toms.

40. A flexible resin composition for medical use according to Claim 37, wherein the acid moiety of said dicarboxylic ester is the residue of maleic acid, succinic acid, glutaric acid, adipic acid, itaconic acid, sebacic acid, dodecandioic acid, or oxalacetic acid.

41. A flexible resin composition for medical use according to Claim 34 or Claim 35,wherein said carboxylic ester compound (A) is selected from the group consisting of

isopropyl 2-ethylhexyl maleate, isopropyl decyl maleate, isopropyl isotridecyl maleate, isopropyl tetradecyl maleate, and isopropyl myristyl maleate.

42. A flexible resin composition for medical use according to Claim 34 or Claim 35, wherein at least one of the monovalent hydrocarbon groups coupled with the ether oxygen of said ether compound (B) possesses a branched structure.

43. A flexible resin composition for medical use according to Claim 34 or Claim 35, wherein said ether compound (B) possesses at least two ether bonds.

44. A flexible resin composition for medical use according to Claim 43, wherein at least two of the monovalent hydrocarbon groups coupled with the ether oxygen possess chains different in length.

45. A flexible resin composition for medical use according to Claim 34 or Claim 35, wherein said ether compound (B) is glycerine diether represented by the general formula (IV') :

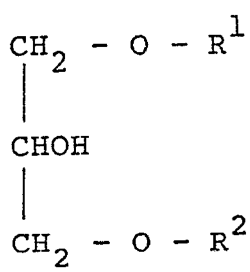

(IV')

wherein $R^1$ and $R^2$ are independently a chain type hydrocarbon group of 3 to 20 carbon atoms.

46. A flexible resin composition for medical use according to Claim 34 or Claim 35, wherein said glycerine diether represented by the general formula (IV') is glycerine-1-butyl-3-isostearyl ether or glycerine-1,3-bis(2-ethylhexyl) ether.

47. A flexible resin composition for medical use according to Claim 34 or Claim 35,wherein at least either of the substituents, R and R', in the general formula (V) of said monocarboxylic ester compound (C) possesses a branched structure.

48. A flexible resin composition for medical use according to Claim 34 or Claim 35, wherein the compound represented by the general formula (V) is isopropyl isolaurate, isopropyl oleate, 2-ethylhexyl isostearate, or 2-ethylhexyl-2-ethylhexanoate.

49. A flexible resin composition for medical use according to Claim 34 or Claim 35, wherein said flexible resin is an internally plasticized vinyl chloride type resin, polyester, polyurethane, ethylene-vinyl acetate copolymer, or a polymer blend of polyvinyl chloride with polyurethane, ethylenic polymer or caprolactone type polymer.

50. A flexible resin composition for medical use according to Claim 49, wherein said internally plasticized vinyl chloride resin is a urethane-vinyl chloride copolymer, a vinyl acetate-vinyl chloride copolymer, or an ethylene-vinyl acetate-vinyl chloride copolymer.

51. A rigid resin composition for medical use, characterized by having at least one hemolysis depressant selected from the group consisting of:

(A) a carboxylic ester compound

i) possessing at least two ester bonds,

ii) having monovalent hydrocarbon groups coupled with the ester bonds, at least two of hydrocarbon groups being chain type hydrocarbon groups differing in number of carbon atoms,

iii) at least one of said monovalent hydrocarbon groups is a hydrocarbon group of not more than 3 or not less than 13 carbon atoms, providing that all of said monovalent hydrocarbon groups are not

hydrocarbon groups of not more than 3 carbon atoms or hydrocarbon groups of not less than 13 carbon atoms, and

iv) at least one of said monovalent hydrocarbon groups possesses a branched structure, and

v) possessing a molecular weight of not more than 1,000;

(B) a ether compound possessing at least one ether bond and chain type monovalent hydrocarbon groups having 3 to 20 carbon atoms coupled with the ether oxygen, the molecular weight of the ether compound being not more than 1,000; and

(C) a monocarboxylic ester compound represented by the general formula(V):

RCOOR' (V)

wherein R and R' are independently a chain type hydrocarbon group of not less than 3 carbon atoms, providing that the total of the carbon atoms or R and R' falls in the range of 11 to 30,

incorporated in a rigid resin composition.

52. A rigid resin composition for medical use according to Claim 51, wherein said hemolysis depressant is incorporated therein in an amount in the range of 0.5 to 5% by weight.

53. A rigid resin composition for medical use according to Claim 51 or Claim 52, wherein said carboxylic ester compound (A) possesses two ester bonds.

54. A rigid resin composition for medical use according to Claim 51 or Claim 52, wherein said carboxylic ester compound (A) is a dicarboxylic ester.

55. A rigid resin composition for medical use according to Claim 51 or Claim 52, wherein one of the monovalent hydrocarbon groups coupled with the ester bonds of said carboxylic ester compound (A) is hydrocarbon group of three carbon atoms and the other monovalent hydrocarbon groups are hydrocarbon groups of 8 to 20 carbon atoms.

56. A rigid resin composition for medical use according to Claim 54, wherein the divalent hydrocarbon groups in acid moiety of said dicarboxylic ester are hydrocarbon groups of 2 to 12 carbon atoms.

57. A rigid resin composition for medical use according to Claim 54, wherein the acid moiety of said dicarboxylic ester is the residue of maleic acid, succinic acid, glytaric acid, adipic acid, itaconic acid, sebacic acid, dodecandioic acid, or oxalacetic acid.

58. A rigid resin composition for medical use according to Claim 51 or Claim 52, wherein said carboxylic ester compound (A) is selected from the group consisting of isopropyl 2-ethylhexyl maleate, isorpopyl decyl maleate, isopropyl isotridecyl maleate, isopropyl tetradecyl maleate, and isopropyl myristyl maleate.

59. A rigid resin composition for medical use according to Claim 51 or Claim 52, wherein at least one of the monovalent hydrocarbon groups coupled with the ether oxygen of said ether compound (B) possesses a branched structure.

60. A rigid resin composition for medical use according to Claim 51 or Claim 52, wherein said ether compound (B) possesses at least two ether bonds.

61. A rigid resin composition for medical use according to Claim 60, wherein at least two of the monovalent hydrocarbon groups coupled with the ether oxygen possess chains different in length.

62. A rigid resin composition for medical use according to Claim 51 or Claim 52, wherein said ether compound (B) is glycerine diether represented by the general formula (IV') :

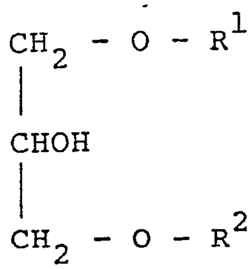

(IV')

wherein $R^1$ and $R^2$ are independently a chain type hydrocarbon group of 3 to 20 carbon atoms.

63. A rigid resin composition for medical use according to Claim 51 or Claim 52, wherein said glycerine diether represented by the general formula (IV') is glycerine-1-butyl-3-isostearyl ether or glycerine-1,3-bis(2-ethylhexyl) ether.

64. A rigid resin composition for medical use according to Claim 51 or Claim 52, wherein at least either of the substituents, R and R',in the general formula (V) of said monocarboxylic ester compound (C) possesses a branched structure.

65. A rigid resin composition for medical use according to Claim 51 or Claim 51, wherein the compound represented by the general formula (V) is isopropyl isolaurate, isopropyl oleate, 2-ethylhexyl isostearate, or 2-ethylhexyl 2-ethylhexanoate.

66. A rigid resin composition for medical use according to Claim 51 or Claim 52, wherein said rigid resin is selected from the group consisting of rigid vinyl chloride type resin, acryl type resin, styrene type resin, olefin type resin, thermoplastic polyester type resin, and polycarbonate.

67. A rigid resin composition for medical use according to Claim 66, wherein said acryl type resin is a homopolymer or a copolymer of methyl methacrylate, methyl acrylate, ethyl methacrylate, ethyl acrylate, acrylonitrile, or methacrylonitrile.

68. A rigid resin composition for medical use according to Claim 66, wherein said styrene type resin is polystyrene, acrylonitrile-styrene copolymer or acrylonitrile-butadiene-styrene copolymer.

69. A rigid resin composition for medical use according to Claim 66, wherein said olefin type resin is polyethylene, polypropylene or ethylene-propyelene copolymer.

70. A rigid resin composition for medical use according to Claim 66, wherein said thermoplastic polyester resin is polyethylene terephthalate or polybutylene terephthalate.

71. A medical implement characterized by being made substantially of a flexible vinyl chloride type resin composition comprising a vinyl chloride type resin, a plasticizer, and at least one hemolysis depressant selected from the group consisting of:

(A) a carboxylic ester compound

i) possessing at least two ester bonds,

ii) having coupled with the ester bonds such monovalent hydrocarbon groups at least two of which are chain type hydrocarbon groups differing in number of carbon atoms,

iii) at least one of said monovalent hydrocarbon groups is a hydrocarbon group of not more than 3 or not less than 13 carbon atoms, providing that all of said monovalent hydrocarbon groups are not hydrocarbon groups of not more than 3 carbon atoms or hydrocarbon groups of not less than 13 carbon atoms, and

iv) at least one of said monovalent hydrocarbon groups possesses a branched structure, and

v) possessing a molecular weight of not more than 1,000;

(B) an ether compound possessing at least one ether bond and having coupled with the ether oxygen of said ether bond such chain type monovalent hydrocarbon groups of 3 to 20 carbon atoms and possessing a molecular weight of not more than 1,000; and

(C) a monocarboxylic ester compound represented by the general formula(V):

RCOOR' (V)

wherein R and R' are independently a chain type hydrocarbon group of not less than 3 carbon atoms, providing that the total of the carbon atoms or R and R' falls in the range of 11 to 30.

72. A medical implement according to Claim 71, which is substantially made of a flexible vinyl chloride type resin composition incorporating therein said plasticizer in an amount in the range of 10 to 45% by weight and said hemolysis depressant in the range of 1 to 30% by weight.

73. A medical implement according to Claim 71 or Claim 72, wherein said carboxylic ester compound (A) possesses two ester bonds.

74. A medical implement according to Claim 71 or Claim 72, wherein said carboxylic ester compound (A) is a dicarboxylic ester.

75. A medical implement according to Claim 71 or Claim 72, wherein one of the monovalent hydrocarbon group coupled with the ester bonds of said carboxylic ester compound (A) is a hydrocarbon group of three carbon atoms and the other monovalent hydrocarbon groups are hydrocarbon groups of 8 to 20 carbon atoms.

76. A medical implement according to Claim 74, wherein the divalent hydrocarbon groups in the acid moiety of said dicarboxylic ester are hydrocarbon groups of 2 to 12 carbon atoms.

77. A medical implement according to Claim 74, wherein the acid moiety of said dicarboxylic ester is the residue of maleic acid, succinic acid, glutaric acid, adipic acid, itaconic acid, sebacic acid, dodecandioic acid, or oxalacetic acid.

78. A medical implement according to Claim 71 or Claim 72, wherein said carboxylic ester compound (A) is selected from the group consisting of isopropyl-2-ethylhexyl maleate, isopropyl decyl maleate, isopropyl isotridecyl maleate, isopropyl tetradecyl maleate, and isorpopyl myristyl maleate.

79. A medical implement according to Claim 71 or Claim 72, wherein at least one of the monovalent hydrocarbon groups coupled with the ether oxygen of said ether compound (B) possesses a branched structure.

80. A medical implement according to Claim 71 or Claim 72, wherein said ether compound (B) possesses at least two ether bonds.

81. A medical implement according to Claim 80, wherein at least two of the monovalent hydrocarbon groups coupled with the ether oxygen possess chains different in length.

82. A medical implement according to Claim 71 or Claim 72, wherein said ether compound (B) is glycerine diether represented by the general formula (IV'):

$$\begin{array}{l} CH_2 - O - R^1 \\ | \\ CHOH \\ | \\ CH_2 - O - R^2 \end{array} \qquad (IV')$$

wherein $R^1$ and $R^2$ are independently a chain type hydrocarbon group of 3 to 20 carbon atoms.

83. A medical implement according to Claim 71 or Claim 72, wherein said glycerine diether represented by the genral formula (IV') is glycerine-1-butyl-3-isostearyl ether or glycerine-1,3-bis(2-ethylhexyl) ether.

84. A medical implement according to Claim 71 or Claim 72, wherein at least either of the substituents, R and R', in the general formula (V) of said monocarboxylic ester compound (C) possesses a branched structure.

85. A medical implement according to Claim 71 or Claim 72, wherein the compound represented by the general formula (V) is isopropyl isolaurate, isopropyl oleate, 2-ethylhexyl isostearate, or 2-ethylhexyl-2-ethylhexanoate.

86. A medical implement according to Claim 71 or Claim 72, wherein said plasticizer possesses a low exuding property.

87. A medical implement according to Claim 71 or Claim 72, wherein said plasticizer is selected from the group consisting of trialkyl trimellitates, dinoraml alkyl phthalate, and tetraalkyl pyromellitates.

88. A medical implement according to Claim 87, wherein said plasticizer is dinormal decyl phthalate.

89. A medical implement according to Claim 87, wherein said plasticizer is trioctyl trimellitate.

90. A medical implement according to Claim 71 or Claim 72, which is a container for blood.

91. A medical implement according to Claim 71 or Claim 72, which is capable of withstanding the impacts of sterilization in an autoclave.

92. A medical implement, characterized by being made substantially of a flexible resin composition having at least one hemolysis depressant selected from the group consisting of:

(A) a carboxylic ester compound

i) possessing at least two ester bonds,

ii) having monovalent hydrocarbon groups coupled with the ester bonds, at least two of hydrocarbon groups being chain type hydrocarbon groups differing in number of carbon atoms,

iii) at least one of said monovalent hydrocarbon groups is a hydrocarbon group of not more than 3 or not less than 13 carbon atoms, providing that all of said monovalent hydrocarbon groups are not hydrocarbon groups of not more than 3 carbon atoms or hydrocarbon groups of not less than 13 carbon atoms, and

iv) at least one of said monovalent hydrocarbon groups possesses a branched structure, and

v) possessing a molecular weight of not more than 1,000;

(B) a ether compound possessing at least one ether bond and chain type monovalent hydrocarbon groups having 3 to 20 carbon atoms coupled with the ether oxygen, the molecular weight of the ether compound being not more than 1,000; and

(C) an monocarboxylic ester compound represented by the general formula(V):

RCOOR' (V)

wherein R and R' are independently a chain type hydrocarbon group of not less than 3 carbon atoms, providing that the total of the carbon atoms or R and R' falls in the range of 11 to 30,

incorporated in a flexible resin composition containing no plasticizer.

93. A medical implement according to Claim 92, which is made substantially of a flexible resin composition incorporating therein said hemolysis depressant in an amount in the rnage of 5 to 35% by weight.

94. A medical implement according to Claim 92 or Claim 93, wherein said carboxylic ester compound (A) possesses two ester bonds.

95. A medical implement according to Claim 92 or Claim 93, wherein said carboxylic ester compound (A) is a dicarboxylic ester.

96. A medical implement according to Claim 92 or Claim 93, wherein one of the monovalent hydrocarbon groups coupled with the ester bonds of said carboxylic ester compound (A) is a hydrocarbon group of three carbon atoms and the other monovalent hydrocarbon groups are hydrocarbon groups of 8 to 20 carbon atoms.

97. A medical implement according to Claim 95, wherein the divalent hydrocarbon groups in the acid moiety of said dicarboylic ester are hydrocarbon groups of 2 to 12 carbon atoms.

98. A medical implement according to Claim 95, wherein the acid moiety of said dicarboxylic ester is the residue of maleic acid, succinic acid, glutaric acid, adipic acid, itaconic acid, sebacic acid, dodecandioic acid, or oxalacetic acid.

99. A medical implement according to Claim 92 or Claim 93, wherein said carboxylic ester compound (A) is selected from the group consisting of isopropyl 2-ethylhexyl maleate, isopropyl decyl maleate, isopropyl isotridecyl maleate, isopropyl tetradecyl maleate, and isopropyl myristyl maleate.

100. A medical implement according to Claim 92 or Claim 93, wherein at least one of the monovalent hydrocarbon groups coupled with the ether oxygen of said ether compound (B) possesses a branched structure.

101. A medical implement according to Claim 92 or Claim 93, wherein said ether compound (B) possesses at least two ether bonds.

102. A medical implement according to Claim 101, wherein at least two of the monovalent hydrocarbon groups coupled with the ether oxygen possess chains different in length.

103. A medical implement according to Claim 92 or Claim 93, wherein said ether compound (B) is glycerine diether represented by the general formula (IV'):

$$\begin{array}{l} CH_2 - O - R^1 \\ | \\ CHOH \\ | \\ CH_2 - O - R^2 \end{array} \qquad (IV')$$

wherein $R^1$ and $R^2$ are independently a chain type hydrocarbon group of 3 to 20 carbon atoms.

104. A medical implement according to Claim 92 or Claim 93, wherein said glycerine diether represented by the general formula (IV') is glycerine-1-butyl-3-isostearyl ether or glycerine-1,3-bis(2-ethylhexyl) ether.

105. A medical implement according to Claim 92 or Claim 93, wherein at least either of the substituents, R and R', in the general formula (V) of said monocarboxylic ester compound (C) possesses a branched structure.

106. A medical implement according to Claim 92 or Claim 93, wherein the compound represented by the general formula (V) is isopropyl isolaurate, isopropyl oleate, 2-ethylhexyl isostearate, or 2-ethylhexyl 2-ethylhexanoate.

107. A medical implement accoring to Claim 92 or Claim 93, wherein said flexible resin is an internally plasticized vinyl chloride type resin, polyester, polyurethane, ethylene-vinyl acetate copolymer, or a polymer blend of polyvinyl chloride with polyurethane, ethylenic polymer or caprolactone type polymer.

108. A medical implement according to Claim 107, wherein said internally plasticized vinyl chloride resin is a urethane-vinyl chloride copolymer, a vinyl acetate-vinyl chloride copolymer, or an ethylene-vinyl acetate-vinyl chloride copolymer.

109. A medical implement according to Claim 92 or Claim 93, which is a container for blood.

110. A medical implement according to Claim 92 or Claim 93, which is capable of withstanding the impacts of sterilization in an autoclave.

111. A medical implement characterized by being made substantially of a rigid resin composition having at least one hemolysis depressant selected from the group consisting of:

(A) a carboxylic ester compound
- i) possessing at least two ester bonds,
- ii) having monovalent hydrocarbon groups coupled with the ester bonds, at least two of hydrocarbon groups being chain type hydrocarbon groups differing in number of carbon atoms,
- iii) at least one of said monovalent hydrocarbon groups is a hydrocarbon group of not more than 3 or not less than 13 carbon atoms, providing that all of said monovalent hydrocarbon groups are not hydrocarbon groups of not more than 3 carbon atoms or hydrocarbon groups of not less than 13 carbon atoms, and
- iv) at least one of said monovalent hydrocarbon groups possesses a branched structure, and
- v) possessing a molecular weight of not more than 1,000;

(B) an ether compound possessing at least one ether bond and chain type monovalent hydrocarbon groups having 3 to 20 carbon atoms coupled with the ether oxygen, the molecular weight of the ether compound being not more than 1,000; and

(C) a monocarboxylic ester compound represented by the general formula(V):

$$RCOOR' \qquad (V)$$

wherein R and R' are independently a chain type hydrocarbon group of not less than 3 carbon atoms, providing that the total of the carbon atoms or R and R' falls in the range of 11 to 30, incorporatd in a rigid resin composition.

112. A medical implement according to Claim 111, which is made substantially of a rigid resin composition for medical use incorporating therein said hemolysis depressant in an amount in the range of 0.5 to 5% by weight.

113. A medical implement according to Claim 111 or Claim 112, wherein said carboxylic ester compound (A) possesses two ester bonds.

114. A medical implement according to Claim 111 or Claim 112, wherein said carboxylic ester compound (A) is a dicarboxylic ester.

115. A medical implement according to Claim 111 or Claim 112, wherein one of the monovalent hydrocarbon groups coupled with the ester bonds of said carboxylic ester compound (A) is a hydrocarbon group of three carbon atoms and the other monovalent hydrocarbon groups are hydrocarbon groups of 8 to 20 carbon atoms.

116. A medical implement according to Claim 114, wherein the divalent hydrocarbon groups in the acid moiety of said dicarboxylic ester are hydrocarbon groups of 2 to 12 carbon atoms.

117. A medical implement according to Claim 114, wherein the acid moiety of said dicarboxylic ester is the residue of maleic acid, succinic acid, glutaric acid, adipic acid, itaconic acid, sebacic acid, dodecandioic acid, or oxalacetic acid.

118. A medical implement according to Claim 112, wherein said carboxylic ester compound (A) is selected from the group consisting of isopropyl 2-ethylhexyl maleate, ispropyl decyl maleate, isopropyl isotridecyl maleate, isopropyl tetradecyl maleate, and ispropyl myristyl maleate.

119. A medcial implement according to Claim 111 or Claim 112, wherein at least one of the monovalent hydrocarbon groups coupled with the ether oxygen of said ether compound (B) possesses a branched structure.

120. A medical implement according to Claim 111 or Claim 112, wherein said ether compound (B) possesses at lest two ether bonds.

121. A medical implement according to Claim 120, wherein at least two of the monovalent hydrocarbon groups coupled with the ether oxygen possess chains different in length.

122. A medical implement according to Claim 111 or Claim 112, wherein said ether compound (B) is glycerine diether represented by the general formula (IV') :

$$\begin{array}{l} CH_2 - O - R^1 \\ | \\ CHOH \\ | \\ CH_2 - O - R^2 \end{array} \qquad (IV')$$

wherein $R^1$ and $R^2$ are independently a chain type hydrocarbon group of 3 to 20 carbon atoms.

123. A medical implement according to Claim 111 or Claim 112, wherein said glycerine diether represented by the general formula (IV') is glycerine-1-butyl-3-isostearyl ether or glycerine-1,3-bis(2-ethylhexyl) ether.

124. A medical implement according to Claim 111 or Claim 112, wherein at least either of the substituents, R and R',in the general formula (V) of said monocarboxylic ester compound (C) possesses a branched structure.

125. A medical implement according to Claim 111 or Claim 112, wherein the compound represented by the general formula (V) is isopropyl isolaurate, isopropyl oleate, 2-ethylhexyl isostearate, or 2-ethylhexyl 2-ethylhexanoate.

126. A medcial implement according to Claim 111 or Claim 112, wherein said rigid resin is selected from the group consisting of rigid vinyl chloride type resin, acryl type resin, styrene type resin, olefin type resin, thermoplastic polyester type resin, and polycarbonate.

127. A medical implement according to Claim 126, wherein said acryl type resin is a homopolymer or a copolymer of methyl methacrylate, methyl acrylate, ethyl methacrylate, ethyl acrylate, acrylonitrile, or methacrylonitrile.

128. A medical implement according to Claim 126, wherein said styrene type resin is polystyrene, acrylonitrile-styrene copolymer or acrylonitrile-butadiene-styrene copolymer.

129. A medical implement according to Claim 126, wherein said olefin type resin is polyethylene, polypropylene or ethylene-propylene copolymer.

130. A medical implement accoding to Claim 126,wherein said thermoplastic polyester resin is polyethylene terephthalate or polybutylene terephthalate.

131. A medical implement according to Claim 111 or Claim 112, which is a blood collection tube.

132. A blood preserving liquid, characterized by incorporating therein at least one hemolysis depressant selected from the group consisting of :

(A) a carboxylic ester compound

i) possessing at least two ester bonds,

ii) having monovalent hydrocarbon groups coupled with the ester bonds, at least two of hydrocarbon groups being chain type hydrocarbon groups differing in number of carbon atoms,

iii) at least one of said monovalent hydrocarbon groups is a hydrocarbon group of not more than 3 or not less than 13 carbon atoms, providing that all of said monovalent hydrocarbon groups are not

hydrocarbon groups of not more than 3 carbon atoms or hydrocarbon groups of not less than 13 carbon atoms, and

iv) at least one of said monovalent hydrocarbon groups possesses a branched structure, and

v) possessing a molecular weight of not more than 1,000;

(B) an ether compound possessing at least one ether bond and chain type monovalent hydrocarbon groups having 3 to 20 carbon atoms coupled with the ether oxygen, the molecular weight of the ether compound being not more than 1,000; and

(C) a monocarboxylic ester compound represented by the general formula(V):

RCOOR' (V)

wherein R and R' are independently a chain type hydrocarbon group of not less than 3 carbon atoms, providing that the total of the carbon atoms or R and R' falls in the range of 11 to 30.

133. A blood preserving liquid according to Claim 132, wherein said carboxylic ester compound (A) possesses two ester bonds.

134. A blood preserving liquid according to Claim 132, wherein said carboxylic ester compound (A) is a dicarboxylic ester.

135. A blood preserving liquid according to Claim 132, wherein one of the monovalent hydrocarbon groups coupled with the ester bonds of said carboxylic ester compound (A) is a hydrocarbon group of three carbon atoms and the other monovalent hydrocarbon groups are hydrocarbon groups of 8 to 20 carbon atoms.

136. A blood preserving liquid according to Claim 134, wherein the divalent hydrocarbon groups in the acid moiety of said dicarboxylic ester are hydrocarbon groups of 2 to 12 carbon atoms.

137. A blood preserving liuqid according to Claim 134, wherein the acid moiety of said dicarboxylic ester is the residue of maleic acid, succinic acid, glutaric acid, adipic acid, itaconic acid, sebacic acid, dodecandioic acid, or oxalacetic acid.

138. A blood preserving liquid according to Claim 132, wherein said carboxylic ester compound (A) is selected from the group consisting of isopropyl 2-ethylhexyl maleate, isopropyl decyl maleate, isopropyl isotridecyl maleate, isopropyl tetradecyl maleate, and isopropyl myristyl maleate.

139. A blood preserving liquid according to Claim 132, wherein at least one of the monovalent hydrocarbon groups coupled with the ether oxygen of said ether compound (B) possesses a branched structure.

140. A blood preserving liquid according to Claim 132, wherein said ether compound (B) possesses at least two ehter bonds.

141. A blood preservig liquid according to Claim 140, wherein at least two of the monovalent hydrocarbon groups coupled with the ether oxygen possess chains different in length.

142. A blood preserving liquid according to Claim 132, wherein said ether compound (B) is glycerine diether represented by the general formula (IV') :

$$\begin{array}{l} CH_2 - O - R^1 \\ | \\ CHOH \\ | \\ CH_2 - O - R^2 \end{array} \qquad (IV')$$

wherein $R^1$ and $R^2$ are independently a chain type hydrocarbon group of 3 to 20 carbon atoms.

143. A blood preserving liquid according to Claim 132, wherein said glycerine diether represented by the general formula (IV') is glycerine-1-butyl-3-isostearyl ether or glycerine-1,3-bis(2-ethylhexyl) ether.

144. A blood preserving liquid according to Claim 132, wherein at least either of the substituents, R and R', in the general formula (V) of said monocarboxylic ester compound (C) possesses a branched structure.

145. A blood preserving liquid according to Claim 132, wherein the compound represented by the general formula (V) is isopropyl isolaurate, isopropyl oleate, 2-ethylhexyl isostearate, or 2-ethylhexyl 2-ethylhexanoate.

146. A blood preserving liquid according to Claim 133, wherein said hemolysis depressant is incorporated in the form of an emulsion.

147. A blood preserving liquid according to Claim 132, wherein said hemolysis depressant is incorporated in the form of an clathrate compound.

148. A blood preserving liquid according to Claim 132, which is an anticoagulant preservative.

149. A blood preserving liquid according to Claim 132, which further comprises at least one compound selected from the group consisting of sodium citrate, citric acid, glucose monosodium phosphate, adenine, sodium chloride, mannitol, maltose, sorbitol, multitol, sucrose and lactose.

150. A blood preserving liquid according to Claim 132, wherein said hemolysis depressant is incorporated in a basic liquid selected from the group consisting of ACD liquid, CPD liquid, CPDA-1 liquid, CPDA-2 liquid, SAG liquid and modified SAG liquid incorporating therein mannitol, maltose, multitol, sorbitol, sucrose, or lactose.

151. A blood preserving liquid according to Claim 132, wherein said hemolysis depressant is incorporated in an amount such as to be contained in a final concentration in the range of 100 μM to 10 mM.

152. A blood preserving liquid according to Claim 132, wherein said hemolysis depressant is incorporated in an amount such as to be contained in a final concentration in the range of 400 μM to 4 mM.

POOR QUALITY

FIG. 1

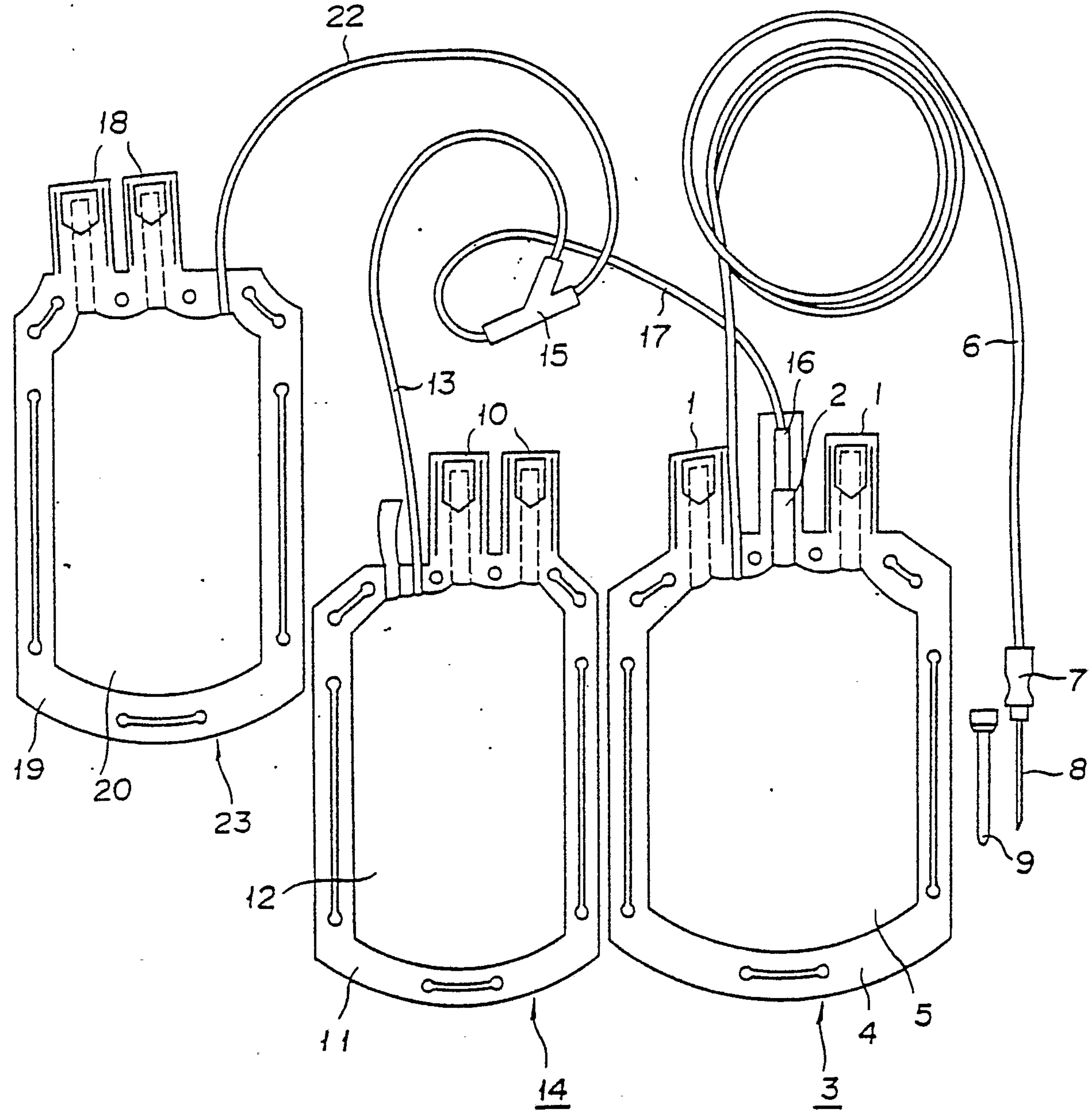

FIG. 2

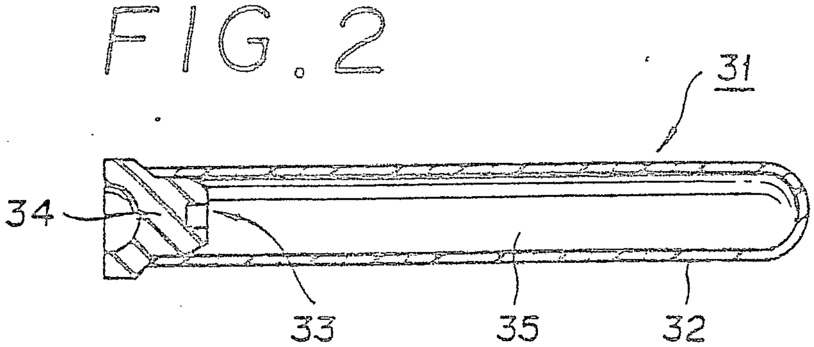


FIG. 3

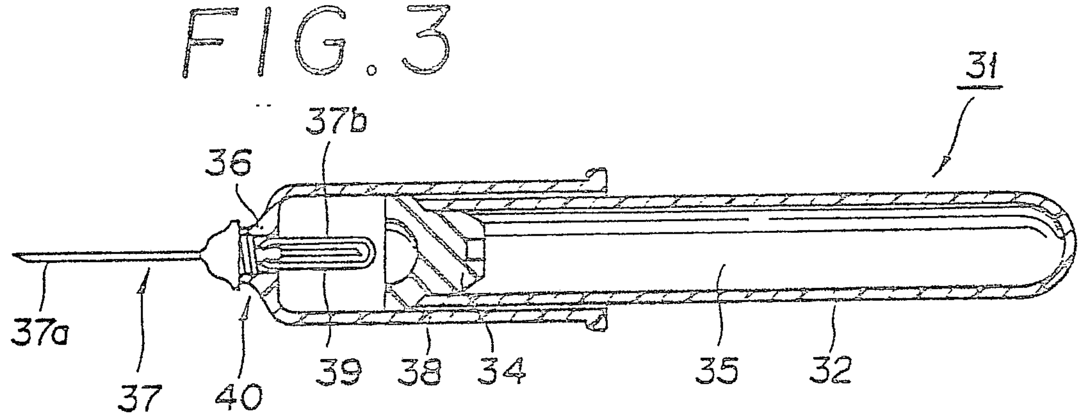


FIG. 4

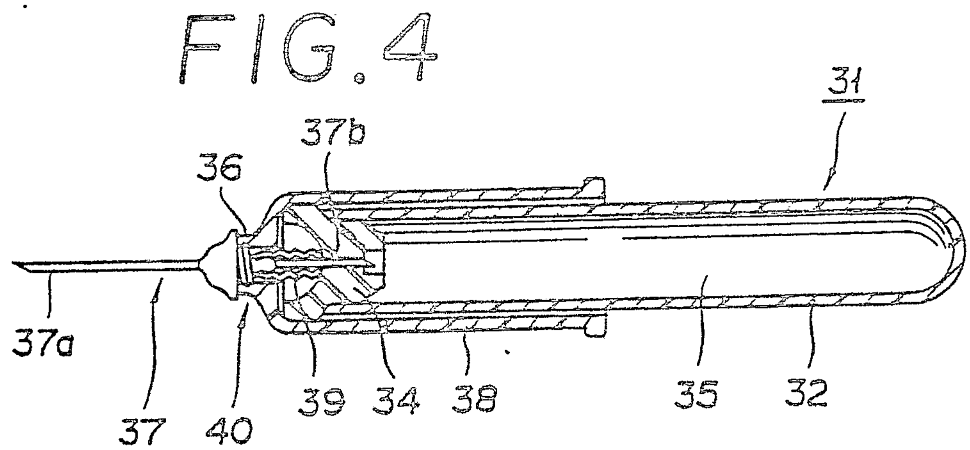


POOR QUALITY

# INTERNATIONAL SEARCH REPORT

0334956

International Application No PCT/JP87/00810

**I. CLASSIFICATION OF SUBJECT MATTER** (if several classification symbols apply, indicate all) [3]

According to International Patent Classification (IPC) or to both National Classification and IPC

Int.Cl[4] A61K35/14, A61L3/00, A61M5/00, A01N1/02

**II. FIELDS SEARCHED**

Minimum Documentation Searched [4]

| Classification System | Classification Symbols |
|---|---|
| IPC | A61K35/14, A61L31/00, A01N1/00 |

Documentation Searched other than Minimum Documentation to the Extent that such Documents are Included in the Fields Searched [5]

**III. DOCUMENTS CONSIDERED TO BE RELEVANT** [14]

| Category * | Citation of Document, [16] with indication, where appropriate, of the relevant passages [17] | Relevant to Claim No. [18] |
|---|---|---|
| A | JP, A, 56-91758 (Baxter Travenol Laboratories Inc.) 24 July 1981 (24. 07. 81) & DE, A1, 3004522, & US, A, 4326025 | 1-152 |
| A | JP, A, 61-2864 (Terumo Kabushiki Kaisha) 8 January 1986 (08. 01. 86) (Family: none) | 1-152 |

* Special categories of cited documents: [15]

"A" document defining the general state of the art which is not considered to be of particular relevance

"E" earlier document but published on or after the international filing date

"L" document which may throw doubts on priority claim(s) or which is cited to establish the publication date of another citation or other special reason (as specified)

"O" document referring to an oral disclosure, use, exhibition or other means

"P" document published prior to the international filing date but later than the priority date claimed

"T" later document published after the international filing date or priority date and not in conflict with the application but cited to understand the principle or theory underlying the invention

"X" document of particular relevance; the claimed invention cannot be considered novel or cannot be considered to involve an inventive step

"Y" document of particular relevance; the claimed invention cannot be considered to involve an inventive step when the document is combined with one or more other such documents, such combination being obvious to a person skilled in the art

"&" document member of the same patent family

**IV. CERTIFICATION**

| Date of the Actual Completion of the International Search [2] | Date of Mailing of this International Search Report [2] |
|---|---|
| January 11, 1988 (11. 01. 88) | January 25, 1988 (25. 01. 88) |
| **International Searching Authority [1]** | **Signature of Authorized Officer [20]** |
| Japanese Patent Office | |